# EUROPÄISCHE PATENTSCHRIFT

(11) **EP 1 851 239 B1**
(45) Veröffentlichungstag und Bekanntmachung des Hinweises auf die Patenterteilung: **21.01.2009**
(21) Anmeldenummer: 06706871.8
(22) Anmeldetag: 10.02.2006
(51) Int. Cl.: C07K 14/115, C12N 15/86, A61K 48/00

(54) **REPLIKATIONSDEFIZIENTE RNA-VIREN ALS IMPFSTOFFE**
REPLICATION-DEFICIENT RNA VIRUSES AS VACCINES
VIRUS ARN A REPLICATION DEFICIENTE EN TANT QUE VACCINS

(30) Priorität: 11.02.2005 DE 102005006388
(43) Veröffentlichungstag der Anmeldung: 07.11.2007
(73) Patentinhaber: MAX-PLANCK-GESELLSCHAFT ZUR FÖRDERUNG DER WISSENSCHAFTEN E.V., 80539 München (DE)
(72) Erfinder: NEUBERT, Wolfgang, J., 86926 Greifenberg (DE); BOSSOW, Sascha, 86152 Augsburg (DE); SCHLECHT, Sabine, 80638 München (DE)
(74) Vertreter: Ricker, Mathias
(86) Internationale Anmeldenummer: PCT/EP2006/001251
(87) Internationale Veröffentlichungsnummer: WO 2006/084746

(56) Entgegenhaltungen:
- EP-A- 1 437 593
- WO-A-20/04113517
- CORTESE CASE K ET AL: "Mutations in domain V of the Sendai virus L polymerase protein uncouple transcription and replication and differentially affect replication in vitro and in vivo" VIROLOGY, Bd. 277, Nr. 2, 25. November 2000 (2000-11-25), Seiten 387-396, XP004435822 ISSN: 0042-6822
- KHATTAR SUNIL K ET AL: "Deletion and substitution analysis defines regions and residues within the phosphoprotein of bovine respiratory syncytial virus that affect transcription, RNA replication, and interaction with the nucleoprotein" VIROLOGY, Bd. 285, Nr. 2, 5. Juli 2001 (2001-07-05), Seiten 253-269, XP002382075 ISSN: 0042-6822
- MYERS TINA M ET AL: "An amino-terminal domain of the Sendai virus nucleocapsid protein is required for template function in viral RNA synthesis" JOURNAL OF VIROLOGY, Bd. 71, Nr. 2, 1997, Seiten 918-924, XP002382076 ISSN: 0022-538X
- CURRAN JOSEPH: "Reexamination of the Sendai virus P protein domains required for RNA synthesis: A possible supplemental role for the protein" VIROLOGY, Bd. 221, Nr. 1, 1996, Seiten 130-140, XP002382077 ISSN: 0042-6822 in der Anmeldung erwähnt

### Sequenzprotokoll

<110> Max-Planck-Gesellschaft zur Förderung der Wissenschaften e.V.
<120> Replikationsdefiziente RNA-Viren als Impstoffe
<130> 33778P WO
<150> DE102005006388.8
   <151> 2005-02-11
<160> 28
<170> PatentIn version 3.3
<210> 1
   <211> 19
   <212> DNA
   <213> künstliche Sequenz
<220>
   <223> Oligonukleotid
<400> 1
   ggaaacagct atgaccatg 19
<210> 2
   <211> 58
   <212> DNA
   <213> künstliche Sequenz
<220>
   <223> Oligonukleotid
<400> 2 ggatcattag taccttgaag cctcgtagat cgcggccgcg tgaactttgg cagcaaag 58
<210> 3
   <211> 57
   <212> DNA
   <213> künstliche Sequenz
<220>
   <223> Oligonukleotid
<400> 3
   ggcttcaagg tactaatgat ccgtagtaag aaaaacttag ggtgaaagta ttccacc 57
<210> 4
   <211> 18
   <212> DNA
   <213> künstliche Sequenz
<220>
<223> Oligonukleotid
<400> 4
   ggtaggtgtc tatgaggc 18
<210> 5
   <211> 44
   <212> DNA <213> künstliche Sequenz
<220>
   <223> Oligonukleotid
<400> 5
   ggaaggaaaa gcggccgccg gcgggatcat acgaggcttc aagg 44
<210> 6
   <211> 57
   <212> DNA
   <213> künstliche Sequenz
<220>
   <223> Oligonukleotid
<400> 6
   cctgtgtttc tgcggccgcc gttcgcgagg ccggcccgtg aactttggca gcaaagc 57
<210> 7
   <211> 44
   <212> DNA
   <213> künstliche Sequenz
<400> 7
   cgcgggcccg gggcggccgc gtcgccacca tggtgagcaa gggc 44
<210> 8
   <211> 26
   <212> DNA
   <213> künstliche Sequenz
<220>
   <223> Oligonukleotid
<400> 8
   gatgcatgct cgagcggccg ctttac 26
<210> 9
   <211> 36
   <212> DNA
   <213> künstliche Sequenz
<220>
   <223> Oligonukleotid
<400> 9
   ggattactat cgccggcggt cgccaccatg gtgagc 36
<210> 10
   <211> 38
   <212> DNA
   <213> künstliche Sequenz
<220>
   <223> Oligonukleotid
<400> 10
   cgctaactgt cgccggcgtt tacttgtaca gctcgtcc 38
<210> 11
   <211> 36
   <212> DNA
   <213> künstliche Sequenz
<220>
   <223> Oligonukleotid
<400> 11
   cggatcaagt ggccggccgt cgccaccatg gtgcgc 36
<210> 12
   <211> 42
   <212> DNA
   <213> künstliche Sequenz
<220>
   <223> Oligonukleotid
<400> 12
   cgcgaatatc ggccggccaa gtctacagga acaggtggtg gc 42
<210> 17
   <211> 17
   <212> DNA
   <213> künstliche Sequenz
<220>
   <223> Oligonukleotid
<400> 17
   ccctgacaca ctccttc 17
<210> 18
   <211> 31
   <212> DNA
   <213> künstliche Sequenz
<220>
   <223> Oligonukleotid
<400> 18
   gcgccgctcg aggcggtaag tgtagccgaa g 31
<210> 19
   <211> 34
   <212> DNA
   <213> künstliche Sequenz
<220>
   <223> Oligonukleotid
<400> 19
   cctgcgctcg agctcatccc gggtgaggca tccc 34
<210> 20
   <211> 30
   <212> DNA
   <213> künstliche Sequenz
<220>
   <223> Oligonukleotid
<400> 20
   ggcgacgcgt cagtctcaca gcctaättcg 30
<210> 21
   <211> 47
   <212> DNA
   <213> künstliche Sequenz
<220>
   <223> Oligonukleotid
<400> 21
   cccccttttt ctcgagatgt cgacccaaga taatcgatca ggtgagg 47
<210> 22
   <211> 46
   <212> DNA
   <213> künstliche Sequenz
<220>
   <223> Oligonukleotid
<400> 22
   tttttccccc ctcgagttac tagttggtca gtgactctat gtcctc 46
<210> 27
   <211> 558
   <212> PRT
   <213> künstliche Sequenz
<220>
   <223> chimäres Protein
<400> 27
   Met Pro Thr Ser Ile Leu Leu Ile Ile Thr Thr Met Ile Met Ala Ser
   1               5                   10                  15
   Phe Cys Gln Ile Asp Ile Thr Lys Leu Gln His Val Gly Val Leu Val
               20                  25                  30
   Asn Ser Pro Lys Gly Met Lys Ile Ser Gln Asn Phe Glu Thr Arg Tyr
   35 40 45
   Leu Ile Leu Ser Leu Ile Pro Lys Ile Glu Asp Ser Asn Ser Cys Gly
   50 55 60
   Asp Gln Gln Ile Lys Gln Tyr Lys Arg Leu Leu Asp Arg Leu Ile Ile
   65 70 75 80
   Pro Leu Tyr Asp Gly Leu Arg Leu Gln Lys Asp Val Ile Val Ser Asn
   85 90 95
   Gln Glu Ser Asn Glu Asn Thr Asp Pro Arg Thr Lys Arg Phe Phe Gly
   100 105 110
   Gly Val Ile Gly Thr Ile Ala Leu Gly Val Ala Thr Ser Ala Gln Ile
   115 120 125
   Thr Ala Ala Val Ala Leu Val Glu Ala Lys Gln Ala Arg Ser Asp Ile
   130 135 140
   Glu Lys Leu Lys Glu Ala Ile Arg Asp Thr Asn Lys Ala Val Gln Ser
   145 150 155 160
   Val Gln Ser Ser Ile Gly Asn Leu Ile Val Ala Ile Lys Ser Val Gln
   165 170 175
   Asp Tyr Val Asn Lys Glu Ile Val Pro Ser Ile Ala Arg Leu Gly Cys
   180 185 190
   Glu Ala Ala Gly Leu Gln Leu Gly Ile Ala Leu Thr Gln His Tyr Ser
   195 200 205
   Glu Leu Thr Asn Ile Phe Gly Asp Asn Ile Gly Ser Leu Gln Glu Lys
   210 215 220
   Gly Ile Lys Leu Gln Gly Ile Ala Ser Leu Tyr Arg Thr Asn Ile Thr
   225 230 235 240
   Glu Ile Phe Thr Thr Ser Thr Val Asp Lys Tyr Asp Ile Tyr Asp Leu
   245 250 255
   Leu Phe Thr Glu Ser Ile Lys Val Arg Val Ile Asp Val Asp Leu Asn
   260 265 270
   Asp Tyr Ser Ile Thr Leu Gln Val Arg Leu Pro Leu Leu Thr Arg Leu
           275                 280                 285
   Leu Asn Thr Gln Ile Tyr Arg Val Asp Ser Ile Ser Tyr Asn Ile Gln
       290                 295                 300
   Asn Arg Glu Trp Tyr Ile Pro Leu Pro Ser His Ile Met Thr Lys Gly
   305                 310                 315                 320
   Ala Phe Leu Gly Gly Ala Asp Val Lys Glu Cys Ile Glu Ala Phe Ser
                   325                 330                 335
   Ser Tyr Ile Cys Pro Ser Asp Pro Gly Phe Val Leu Asn His Glu Met
               340                 345                 350
   Glu Ser Cys Leu Ser Gly Asn Ile Ser Gln Cys Pro Arg Thr Val Val
           355                 360                 365
   Lys Ser Asp Ile Val Pro Arg Tyr Ala Phe Val Asn Gly Gly Val Val
       370                 375                 380
   Ala Asn Cys Ile Thr Thr Thr Cys Thr Cys Asn Gly Ile Gly Asn Arg
   385                 390                 395                 400
   Ile Asn Gln Pro Pro Asp Gln Gly Val Lys Ile Ile Thr His Lys Glu
                   405                 410                 415
   Cys Asn Thr Ile Gly Ile Asn Gly Met Leu Phe Asn Thr Asn Lys Glu
               420                 425                 430
   Gly Thr Leu Ala Phe Tyr Thr Pro Asn Asp Ile Thr Leu Asn Asn Ser
           435                 440                 445
   Val Ala Leu Asp Pro Ile Asp Ile Ser Ile Glu Leu Asn Lys Ala Lys
       450                 455                 460
   Ser Asp Leu Glu Glu Ser Lys Glu Trp Ile Arg Arg Ser Asn Gln Lys
   465                 470                 475                 480
   Leu Asp Ser Ile Gly Asn Trp His Gln Ser Ser Thr Thr Val Ile Thr
                   485                 490                 495
   Ile Ile Val Val Met Val Val Ile Leu Val Val Ile Ile Val Ile Val
   500 505 510
   Ile Val Leu Tyr Arg Leu Lys Arg Ser Met Leu Met Gly Asn Pro Asp
   515 520 525
   Asp Arg Ile Pro Arg Asp Thr Tyr Thr Leu Glu Pro Lys Ile Arg His
   530 535 540
   Met Tyr Thr Asn Gly Gly Phe Asp Ala Met Ala Glu Lys Arg
   545 550 555
<210> 28
   <211> 579
   <212> PRT
   <213> künstliche Sequenz
<220>
   <223> chimäres Protein
<400> 28
   Met Asp Gly Asp Arg Gly Lys Arg Asp Ser Tyr Trp Ser Thr Ser Pro
   1 5 10 15
   Ser Gly Ser Thr Thr Lys Leu Ala Ser Gly Trp Glu Arg Ser Ser Lys
   20 25 30
   Val Asp Thr Trp Leu Leu Ile Leu Ser Phe Thr Gln Trp Ala Leu Ser
   35 40 45
   Ile Ala Thr Val Ile Ile Cys Ile Ile Ile Ser Ala Asn Ser Ile Lys
   50 55 60
   Ser Glu Lys Ala His Glu Ser Leu Leu Gln Asp Val Asn Asn Glu Phe
   65 70 75 80
   Met Glu Val Thr Glu Lys Ile Gln Met Ala Ser Asp Asn Ile Asn Asp
   85 90 95
   Leu Ile Gln Ser Gly Val Asn Thr Arg Leu Leu Thr Ile Gln Ser His
   100 105 110
   Val Gln Asn Tyr Ile Pro Ile Ser Leu Thr Gln Gln Met Ser Asp Leu
   115 120 125
   Arg Lys Phe Ile Ser Glu Ile Thr Ile Arg Asn Asp Asn Arg Glu Val
       130                 135                 140
   Pro Pro Gln Arg Ile Thr His Asp Ala Gly Ile Lys Pro Leu Asn Pro
   145                 150                 155                 160
   Asp Asp Phe Trp Arg Cys Thr Ser Gly Leu Pro Ser Leu Met Lys Thr
                   165                 170                 175
   Pro Lys Ile Arg Leu Met Pro Gly Pro Gly Leu Leu Ala Met Pro Thr
               180                 185                 190
   Thr Val Asp Gly Cys Val Arg Thr Pro Ser Leu Val Ile Asn Asp Leu
           195                 200                 205
   Ile Tyr Ala Tyr Thr Ser Asn Leu Ile Thr Arg Gly Cys Gln Asp Ile
       210                 215                 220
   Gly Lys Ser Tyr Gln Val Leu Gln Ile Gly Ile Ile Thr Val Asn Ser
   225                 230                 235                 240
   Asp Leu Val Pro Asp Leu Asn Pro Arg Ile Ser His Thr Phe Asn Ile
                   245                 250                 255
   Asn Asp Asn Arg Lys Ser Cys Ser Leu Ala Leu Leu Asn Thr Asp Val
               260                 265                 270
   Tyr Gln Leu Cys Ser Thr Pro Lys Val Asp Glu Arg Ser Asp Tyr Ala
           275                 280                 285
   Ser Ser Gly Ile Glu Asp Ile Val Leu Asp Ile Val Asn His Asp Gly
       290                 295                 300
   Ser Ile Ser Thr Thr Arg Phe Lys Asn Asn Asn Ile Ser Phe Asp Gln
   305                 310                 315                 320
   Pro Tyr Ala Ala Leu Tyr Pro Ser Val Gly Pro Gly Ile Tyr Tyr Lys
                   325                 330                 335
   Gly Lys Ile Ile Phe Leu Gly Tyr Gly Gly Leu Glu His Pro Ile Asn
               340                 345                 350
   Glu Asn Ala Ile Cys Asn Thr Thr Gly Cys Pro Gly Lys Thr Gln Arg
           355                     360                     365
   Asp Cys Asn Gln Ala Ser His Ser Pro Trp Phe Ser Asp Arg Arg Met
       370                     375                     380
   Val Asn Ser Ile Ile Val Val Asp Lys Gly Leu Asn Ser Ile Pro Lys
   385                     390                     395                     400
   Leu Lys Val Trp Thr Ile Ser Met Arg Gln Asn Tyr Trp Gly Ser Glu
                   405                     410                     415
   Gly Arg Leu Leu Leu Leu Gly Asn Lys Ile Tyr Ile Tyr Thr Arg Ser
               420                     425                     430
   Thr Ser Trp His Ser Lys Leu Gln Leu Gly Ile Ile Asp Ile Thr Asp
           435                     440                     445
   Tyr Ser Asp Ile Arg Ile Lys Trp Thr Trp His Asn Val Leu Ser Arg
       450                     455                     460
   Pro Gly Asn Asn Glu Cys Pro Trp Gly His Ser Cys Pro Asp Gly Cys
   465                     470                     475                     480
   Ile Thr Gly Val Tyr Thr Asp Ala Tyr Pro Leu Asn Pro Thr Gly Ser
                   485                     490                     495
   Ile Val Ser Ser Val Ile Leu Asp Ser Gln Lys Ser Arg Val Asn Pro
               500                     505                     510
   Val Ile Thr Tyr Ser Thr Ser Thr Glu Arg Val Asn Glu Leu Ala Ile
           515                     520                     525
   Arg Asn Lys Thr Leu Ser Ala Gly Tyr Thr Thr Thr Ser Cys Ile Thr
       530                     535                     540
   His Tyr Asn Lys Gly Tyr Cys Phe His Ile Val Glu Ile Asn His Lys
   545                     550                     555                     560
   Ser Leu Asp Thr Phe Gln Pro Met Leu Phe Lys Thr Glu Ile Pro Lys
                   565                     570                     575
   Ser Cys Ser

## Beschreibung

Die vorliegende Erfindung betrifft ein replikationsdefizientes und transkriptionskompetentes Negativstrang RNA-Virus, welches zur Expression von Transgenen und insbesondere für den Bereich der lmpfstoffentwicklung eingesetzt werden kann.

Immunisierungen mit Lebendvakzinen ahmen die natürliche Infektion nach und erzeugen eine umfassende Immunantwort. Bei der Impfung werden abgeschwächte, aber vermehrungsfähige Viren eingesetzt. Die Vermehrung der Impfviren muss so langsam erfolgen, dass eine immunologische Antwort und damit Kontrolle der Vermehrung bzw. Eliminierung des Virus gewährleistet ist. Das Konzept der Lebendvakzine hat sich bei verschiedenen Altersgruppen vielfach bewährt. Allerdings gibt es wichtige Zielgruppen, bei denen lmmunisierungen mit Lebendvakzinen problematisch sind und zusätzliche Sicherungsmaßnahmen erfordern Maternale Antikörper schützen Säuglinge in den ersten Lebensmonaten. Sie stellen gleichzeitig eine Barriere dar, die bei der Lebendimmunisierung überwunden werden muss, ohne dass es dabei andererseits zu einer überschießenden Vermehrung des Impfvirus und den damit verbundenen Impfschäden kommen darf. Eine weitere Zielgruppe stellen ältere Menschen dar, deren Immunsystem nicht mehr so leistungsfähig ist, so dass es zu einer Überlastung durch die Impfung und zu lmpfschäden durch erhöhte Vermehrung des Impfvirus kommen kann. Es ergibt sich also das Problem, die immunologisch hervorragende Lebendimpfung für die Anwendung in bestimmten Zielgruppen noch sicherer zu machen, aber auch für die allgemeine Anwendung das Sicherheitsprofil zu erhöhen.

Negativstrang RNA-Viren, z.B. wie etwa das Tollwutvirus oder das Sendaivirus (SeV), können seit einigen Jahren durch reverse Gentechnik zielgerichtet verändert werden. EP-A-0 702 085 beschreibt die Herstellung von rekombinanten, infektiösen, replizierenden nicht segmentierten Negativstrang RNA-Viren aus klonierter cDNA. EP-A-0 863 202 beschreibt ein rekombinantes Sendaivirus, in dessen Genom ein heterologes Gen inseriert oder ein Gen deletiert oder inaktiviert ist, dessen Genomreplikation aber noch intakt ist.

Als Vakzine-Rückgrat eignen sich Negativstrang RNA-Viren besonders, da ihre Vermehrung im Zytoplasma auf RNA-Niveau abläuft und Gene innerhalb des viralen Genoms einfach ausgetauscht werden können. So ist es bereits erfolgreich gelungen, rekombinante Viren mit Oberflächenproteinen verschiedener Virustypen herzustellen und als Vakzine in Tierversuchen einzusetzen (Schmidt et al., J. Virol. 75 (2001), 4594-4603 und WO 01/42445). Durch den rekombinanten Einbau von F-und HN-Proteinen des humanen Parainfluenzavirus Typ 3 (HPIV 3) und des G- oder F-Proteins des Respiratorischen Syncytial Virus (RSV) in einen Vektor auf Basis des bovinen Parainfluenzavirus Typ 3 (BPIV 3) konnte nach Applikation in Hamstern eine mukosale Immunantwort gegen HPIV 3 und RSV nachgewiesen werden. Eine bivalente Antigenität dieses im Tierversuch getesteten Lebendimpfstoffs ist somit bereits erreicht.

Wegen der Einschaltung der Speziesbarriere soll dieses bovine Parainfluenzavirus mit humanen PIV 3- und RSV-Oberflächenantigenen bereits für eine Anwendung im Humanbereich ausreichend attenuiert sein. Reversionen zum Wildtyp sind nicht zu erwarten, da komplette Gene für virale Oberflächenproteine ausgetauscht wurden. Es wurde bereits mit der klinischen Prüfung der Vakzine begonnen.

Da die beschriebenen Virus-Mutanten aber replikationskompetent sind, kommt es im lmptling sicher zu einer Virusvermehrung, deren Intensität zwar durch die Modifikation des Virus abgeschwächt, aber nicht vollständig ausgeschaltet ist. Die Stärke der zu erwartenden Virämie und damit die Belastung des lmpflings mit Nebenwirkungen hängen dabei von individuellen Faktoren ab.

Im Rahmen der vorliegenden Erfindung sollte versucht werden, die Gefährdungen der Lebendvakzinierung, insbesondere die Gefährdung von Impflingen aus bestimmten Zielgruppen substanziell zu verringern.

Ein Ansatz hierzu besteht in der Unterdrückung der viralen Genomreplikation nach Applikation der Vakzine. Dadurch kann es unabhängig von der immunologischen Leistungsfähigkeit des Impflings nicht zu einer Vermehrung des Virus und entsprechenden Impfschäden kommen.

Bei diesem Ansatz besteht die prinzipielle Schwierigkeit, dass die virale RNA-Polymerase zwei Funktionen, die Synthese viraler mRNA und die Vermehrung der viralen Genome, durchführt. Diese Kopplung muss in der neuen Vakzine aufgehoben sein, da die Vakzine nur noch zur Synthese von viraler mRNA in der Lage sein darf.

Ein weiteres Problem besteht darin, dass das rekombinante Virus eine effiziente Synthese von viraler mRNA bewirken muss, um überhaupt als Lebendvakzine geeignet zu sein. Es bestehen somit zwei sich prinzipiell widersprechende Anfoderungen, welche erhebliche Schwierigkeiten bei der Herstellung von sicheren, aber effizienten Lebendvakzinen auf Basis von Negativstrang RNA-Viren erwarten lassen.

Shoji et al. (Virology 318 (2004), 295-305) beschreiben die Herstellung und Charakterisierung eines P-Gen defizienten Tollwutvirus. Die Herstellung des Virus erfolgt mittels P-Protein exprimierenden Helferzellen. Die Viren sind ohne de novo-Synthese von P-Protein nur zur Primärtranskription in der Lage. Die geringe virale Genexpression zeigt sich in einem sehr schwachen Signal für N-Protein in der Immunfluoreszenz und nur bei sehr wenigen Zellen, ein überzeugenderer Nachweis dieser geringen viralen Genexpression wird erst durch PCR-Analyse geführt. Der Einsatz dieser Virusmutante in einem Challenge-Versuch im Mausmodell soll eine Protektion zeigen, allerdings fehlt ein Kontrollexperiment mit transkriptionsinaktivem Virus und das Intervall für den viralen Challenge ist zu kurz bemessen. Die Dauer der vermeintlichen Protektion wird nicht untersucht Der. Einsatz solcher Virusmutanten für die Entwicklung eines attenuierten Tollwutvakzins erscheint somit wenig erfolgversprechend.

Im Rahmen der zur vorliegenden Erfindung führenden Untersuchungen wurde gefunden, dass bei Paramyxoviren eine Entkopplung der Funktionen. Replikation und Transkription dadurch erreicht werden kann, dass die Bestandteile der Polymerase, die für die Genomreplikationsfunktion essentiell sind, teilweise entfernt werden. Es kann sich hierbei um das virale Protein P handeln oder um eine spezielle funktionelle Domäne eines solchen Proteins.

Überraschenderweise wurde gefunden, dass durch Mutationen, bei denen die Funktion der von dem viralen Gen P kodierten Proteine nicht verständig, sondern teilweise deletiert wird, replikationsdefiziente RNA-Viren hergestellt werden können, die eine ausreichende Transkriptionsfunktion besitzen, um zur Herstellung von Lebendvakzinen geeignet zu sein.

Ein Gegenstand der vorliegenden Erfindung ist somit ein rekombinantes Negativstrang RNA-Virus, das replikationsdefizient und transkriptionskompetent ist. Das erfindungsgemäße Virus enthält ein virales Genom mit einer Mutation in dem Gen P, wobei die Mutation zum Verlust der Genom-Replikation ohne Verlust der sekundären Transkriptionsfähigkeit führt.

Das erfindungsgemäße Virus ist eine Voraussetzung zur Herstellung von Lebendvakzinen, insbesondere zur Herstellung von Lebendvakzinen mit erhöhtem Sicherheitsprofil, die zur Anwendung bei Risikopatienten mit schwachem oder geschädigtem Immunsystem besonders geeignet ist.

Die Erfindung betrifft auch ein Nukleokapsid des rekombinanten Virus, umfassend die virale Negativstrang RNA, komplexiert mit den Proteinen N, L und P sowie die Negativstrang RNA des rekombinanten Virus in isolierter Form.

Ein weiterer Gegenstand der Erfindung ist eine cDNA, die für eine erfindungsgemäße Negativstrang RNA, insbesondere eine virale RNA oder/und eine dazu komplementäre RNA, kodiert.

Noch ein weiterer Gegenstand der Erfindung ist eine Zelllinie zur Vermehrung des erfindungsgemäßen rekombinanten Negativstrang RNA Virus.

Das erfindungsgemäße rekombinante Negativstrang RNA-Virus ist durch Mutation eines Ausgangsvirus in dem Gen P erhältlich. Das Ausgangsvirus kann ein natürliches Negativstrang RNA-Virus, insbesondere aus den Familien *Paramyxoviridae* oder *Rhabdoviridae* oder rekombinanten Varianten davon sein. Besonders bevorzugte Vertreter sind Paramyxoviren, zB. Sendaivirus, humanes oder bovines Parainfluenzavirus, z.B. humanes Parainfluenzavirus (hPIV) Typ 1, 2, 3, 4a oder 4b, Newcastle Disease-Virus, Mumpsvirus, Masernvirus oder humanes Respiratorisches Synctialvirus (hRSV) oder Rhabdoviren, z.B. Vesikuläres Stomatitis-Virus (VSV). Besonders bevorzugt ist das Virus ein Sendaivirus, zB. der Stamm Fushimi (ATCC VR105). Weiterhin von der Erfindung erfasst wurden auch rekombinante Varianten der zuvor genannten Viren, wie sie zB. in EP-A 702 085, EP-A-0 863 202 oder WO 01/42445 beschrieben sind.

Weitere bevorzugte Negativstrang RNA-Viren sind Vertreter der *Rhabdoviridae, Filoviridae, Bomaviridae, Arenaviridae* oder *Bunyaviridae,* zB. VSV.

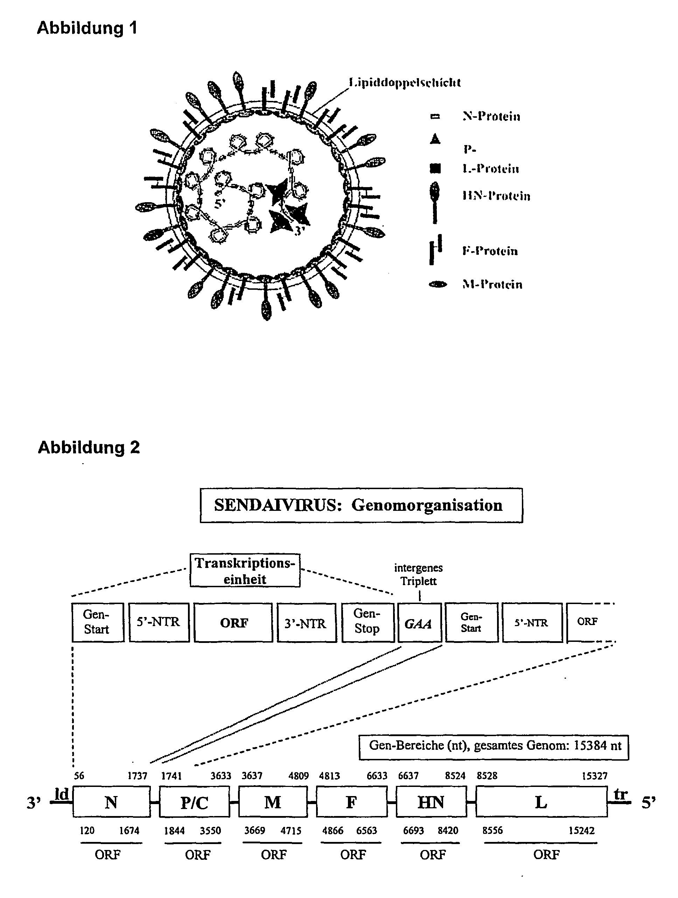

Das Sendaivirus ist - ebenso wie andere Paramyxoviren - ein behülltes Virus mit helikalem Nukleokapsid (Abbildung 1). Die Hülle besteht aus einer Lipidmembran, welche von der Plasmamembran der Wirtszelle stammt, aus welcher das Virus freigesetzt wurde. In die virale Hülle sind transmembrane Glycoproteine verankert, nämlich das Fusionsprotein (F) und die Hämagglutinin-Neuramidase (HN). Das Matrixprotein (M) kleidet die Innenseite der Membran aus. Das in der Hülle enthaltene Nukleokapsid besteht aus einzelsträngiger RNA im Komplex mit Nukleoprotein (N), wobei jeweils 6 Nukleotide der RNA von einem N-Protein gebunden werden, einer RNA-abhängigen RNA-Polymerase (L) und dem Cofaktor Phosphoprotein (P).

Das Negativstrang RNA-Genom des Sendaivirus enthält die Gene der 6 Strukturproteine in der Reihenfolge: 3'-N-P/C-M-F-HN-L-5' (Abbildung 2). Das P/C-Gen kodiert für insgesamt 8 Proteine, das strukturelle Phosphoprotein und alle bisher bekannten Nichtstrukturproteine.

Die Proteine P, N und L sind für eine funktionelle Transkription und Replikation wesentlich (Lamb et al., Paramyxoviridae: The Viruses and their Replication. Fields Virology, 4. Ausgabe (2001), Lippincott, Williams & Wilkins, Philadelphia, 1305-1340).

Das erfindungsgemäße rekombinante Negativstrang RNA-Virus enthält eine Mutation in dem Gen P. Die Mutation kann eine Deletion, Substitution oder/und insertion in dem Gen P sein, die eine Replikationsdefizienz des Virus bewirkt, die aber die Fähigkeit zur Transkription nicht zerstört. Die Mutation betrifft vorzugsweise eine Teilsequenz der von dem Gen P kodierten Proteine, die für die Replikation notwendig ist, während andere für die Transkription notwendige Teilsequenzen funktionell erhalten bleiben.

In der Erfindung weist das rekombinante Virus eine Mutation in Gen P auf und zwar in einer N-terminalen Teilsequenz des Gens P. Die Mutation betrifft vorzugsweise zumindest den Bereich der Aminosäuren 33-41 des Proteins P, die für die Replikationsfähigkeit wesentlich sind. Weiterhin ist bevorzugt, dass der C-terminale Bereich (ab Aminosäure 320) keine die Transkripfionsfunktion beeinträchtigende Mutationen aufweist Besonders bevorzugt ist die Mutation eine zum Verlust der Replikationsfähigkeit führende Mutation im Bereich der Aminosäuren 2-77, beispielsweise eine Deletion von (a) den Aminosäuren 2-77 des vom Gen P kodierten Proteins oder (b) einer Teilsequenz zum Verlust der Replikationsfähigkeit ausreichenden Teilsequenz von (a). Entsprechende Mutationen können auch in P-Proteinen von anderen Negativstrang RNA-Viren, z.B. von anderen Paramyxoviren, z.B. hPIV3, erfolgen.

Das erfindungsgemäße rekombinante Virus ist neptikationsdefizient und transkriptionskompetent Der Verlust der Replikationsfähigkeit bedeutet, dass in einer Zielzelle (einer Zelle, die keine der durch Mutation deletierten Funktionen *in trans* bereitstellt) keine nachweisbare Virusgenom-Vermehrung gefunden wird, wobei im Unterschied zu einer verringerten bzw. konditionalen Replikationsdefizienz auch keine permissiven Bedingungen existieren, unter denen eine Replikation stattfinden kann. Die Bestimmung des Verlusts der Replikationsfähigkeit kann wie in Beispiel 8 beschrieben erfolgen. Das erfindungsgemäße Virus ist jedoch in der Lage, nach Infektion in einer Zielzelle die von ihm kodierten Genprodukte zu transkribieren, so dass eine Expression der viralen Proteine einschließlich eines oder mehrerer heterologer Genprodukte in der Zielzelle erfolgen kann. Wesentlich ist, dass das erfindungsgemäße rekombinante Virus die Fähigkeit zur sekundären Transkription besitzt, d.h. die viralen Genprodukte, die durch primäre Transkription mit den ursprünglich im Nukleokapsid enthaltenen Proteinkomponenten entstehen, sind in der Lage, selbst eine sekundäre Transkription zu bewirken oder/und zu unterstützen. Das Ausmaß der sekundären Transkription führt dabel zu einer Proteinsynthese von vorzugsweise mindestens 1 %, mindestens 2 %, mindestens 3 %, mindestens 4 % oder mindestens 5 % gegenüber einem entsprechenden Wildtypvirus, d.h. einem Virus ohne die Mutation in dem Gen P. Die Fähigkeit zur sekundären Transkription kann gegenüber dem entsprechenden Wildtypvirus verringert sein, vorzugsweise jedoch höchstens um den Faktor 20, besonders bevorzugt höchstens um den Faktor 10. Die Fähigkeit zur sekundären Transkription kann wie in Beispiel 7.1 oder/und 7.3 durch quantitative Bestimmung der Expression eines heterologen Genprodukts, z.B. eines Reporterproteins, ermittelt werden.

Neben der Mutation enthält das erfindungsgemäße rekombinante Virus vorzugsweise mindestens ein Transgen, d.h. mindestens eine für ein heterologes Genprodukt kodierende Sequenz. Das heterologe Genprodukt kann ein Protein, beispielsweise ein Reporterprotein, z.B. ein Fluoreszenzprotein wie etwa GFP oder ein Derivat davon, oder ein Antigen, gegen das eine Immunantwort erzeugt werden soll, oder ein therapeutisches Protein, z.B. ein Protein für die Virotherapie oder auch ein funktionelles RNA-Molekül, z.B. eine Antisense RNA, ein Ribozym oder ein zur RNA-Interferenz befähigtes siRNA-Molekül sein. Bevorzugt ist das heterologe Genprodukt ein Antigen, das von einem Pathogen, wie etwa einem Virus, einem Bakterium, einem Pilz oder einem Protozoon stammt, ein Tumorantigen oder ein Autoantigen. Besonders bevorzugt ist das Antigen ein virales Antigen, das von einem heterologen Negativstrang RNA-Virus, wie etwa einem humanen Parainfluenzavirus oder RSV stammt, z.B. hPIV3 F und HN bzw. hRSV F und G. Das erfindungsgemäße Virus kann ein oder mehrere, z.B. zwei oder drei, für ein heterologes Genprodukt kodierende Sequenzen enthalten.

Für heterologe Genprodukte kodierende Sequenzen können in das Genom des rekombinanten Virus inseriert werden. Andererseits können homologe Genprodukte kodierende Sequenzen, z.B. die Gene F oder/und HN, auch durch Sequenzen substituiert werden, die für heterologe Genprodukte, z.B. chimäre Genprodukte, kodieren. Auch Kombinationen von inserierten und substituierten Transgenen sind möglich.

So können beispielsweise Sequenzen eines Sendaivirus durch heterologe Sequenzen anderer Negativstrang RNA-Viren vollständig oder teilweise ersetzt werden, z.B. durch Sequenzen von Parainfluenzaviren, z.B. hPIV3, oder/und durch Sequenzen von RSV. Besonders bevorzugt werden chimäre Sequenzen verwendet, d.h. Sequenzen, die aus Abschnitten des Basisvirusgenoms und aus Abschnitten eines heterologen Virusgenoms bestehen. Beispielsweise können chimäre Gene F oder/und HN in das Virusgenom eingesetzt werden, die aus Sequenzen des Basisvirusgenoms, z.B. Sendaivirus und aus heterologen Sequenzen, z.B. aus humanen Parainfluenzaviren wie etwa hPIV3, oder/und RSV bestehen.

Das rekombinante Virus kann eine oder mehrere verschiedene Transgene enthalten. Wenn mehrere Transgene vorhanden sind, können diese von gleicher oder verschiedener Herkunft sein, die z.B. aus einem einzigen oder aus mehreren verschiedenen Pathogenen, z.B. Viren, stammen können. So können Transgene aus mehreren, z.B. 2 oder 3 verschiedenen Pathogenen, vorzugsweise Viren, besonders bevorzugt Negativstrang RNA-Viren, vorhanden sein.

Der Einbau von Transgenen in Paramyxoviren ist beispielsweise bei Hasan et al. (J. Gen. Virol. 78 (1997), 2813-2820), Bukreyev et al., (J. Virol. 70 (1996), 6634-6641), Yu et al. (Genes Cells 2 (1997), 457-466), Masaki et al. (FASEBVB J. 15 (2001), 1294-1296), Shiotani et al. (Gene Therapy 8 (2001), 1043-1050) und Bitzer et al. (Mol. Therapy 7 (2003), 210-217) beschrieben. Vorzugsweise werden die Transgene in den 3'-Bereich des viralen Genoms inseriert. Die Insertion erfolgt beispielsweise in Form von Transkriptionskassetten, wobei auf Vektorebene (d.h. auf Ebene des Vektors, z.B. eines Plasmid-Vektors, der für die Negativstrang RNA kodiert) direkt nach dem leader-Bereich eine oder mehrere Transkriptionskassetten mit singulären Restriktionsschnittstellen für die Integration der jeweiligen Leserahmen eingebracht werden. Eine Integration von mehreren Transgenen erfolgt vorzugsweise in jeweils unabhängigen Transkriptionskassetten. Eine Transkriptionskassette enthält vorzugsweise die für das heterologe Genprodukt kodierende Sequenz in operativer Verknüpfung mit einer Transkriptionsstartsequenz und einer Transkriptionsterminationssequenz und vorzugsweise Translationssignalen.

Ein weiterer Gegenstand der vorliegenden Erfindung ist ein einzelsträngiges oder doppelsträngiges DNA-Molekül, z.B. ein cDNA-Molekül, das für ein erfindungsgemäßes rekombinantes Negativstrang RNA-Virusgenom oder eine Vorstufe davon oder das Virus-Antigenom oder einer Vorstufe davon kodiert. Der Begriff "Vorstufe" bedeutet in diesem Zusammenhang, dass das DNA-Molekül noch keine für ein heterologes Genprodukt kodierende Sequenz, sondern lediglich eine Klonierungsstelle zur Einführung einer solchen Sequenz enthält. Die Klonierungsstelle kann eine Restriktionsschnittstelle, beispielsweise eine singuläre oder nicht-singuläre Restriktionsschnittstelle in der DNA oder eine multiple Klonierungsstelle, enthaltend mehrere aufeinander folgende Restriktionsschnittstellen, vorzugsweise singuläre Restriktionsschnittstellen sein. Das für das Virusgenom oder/und die komplementäre Sequenz kodierende DNA-Molekül liegt vorzugsweise in operativer Verknüpfung mit geeigneten Expressionskontrollsequenzen vor.

Das DNA-Molekül ist vorzugsweise ein Vektor, beispielsweise ein Plasmid-Vektor, der zur Propagation in einer geeigneten Wirtszelle, d.h. in einer Vektor- oder Plasmid-Vermehrungszelle, vorzugsweise in einer prokaryontischen Zelle, aber auch in einer eukaryontischen Zelle, insbesondere in einer Säugerzelle, geeignet ist und die hierfür notwendigen genetischen Elemente, wie etwa Replikationsursprung, Integrationssequenzen oder/und Selektionsmarker-Sequenzen, aufweist.

Das DNA-Molekül enthält die für das rekombinante Virus oder die komplementäre Sequenz kodierende Sequenz, vorzugsweise unter Kontrolle eines Transkriptionssignals, so dass bei Transkription mit einer DNAabhängigen RNA-Polymerase in einer zur initialen Herstellung des Virus geeigneten Wirtszelle, d.h. in einer Virus-Herstellungszelle, die virale Negativstrang RNA gebildet werden kann. Das Transkriptionssignal wird so gewählt, dass es in der jeweils verwendeten Wirtszelle eine effiziente Transkription der DNA ermöglicht Hierzu kann auch ein für die jeweilige Zelle heterologes Transkriptionssignal, z.B. ein Bakteriophagenpromotor, wie etwa der T7- oder SP6-Promotor, verwendet werden, wobei die Virus-Herstellungszelle dann auch eine entsprechende heterologe DNAabhängigen RNA-Polymerase, z.B. der T7- oder SP6-RNA-Polymerase, enthalten muss, welche die Transkription bewirkt. Weiterhin enthält das DNA-Molekül neben dem Transkriptionssignal vorzugsweise am 3'-Ende der für das rekombinante Virus kodierenden Sequenz einen Transkriptionsterminator und eine Ribozymsequenz, die eine Spaltung des Transkripts am Ende der viralen Sequenz ermöglicht. Die Virus-Herstellungszelle ist vorzugsweise eine eukaryontische Zelle und insbesondere eine Säugerzelle.

Neben der für das replikationsdefiziente Paramyxovirus kodierenden DNA enthält die erfindungsgemäße Virus-Herstellungszelle darüber hinaus Helfersequenzen, deren Genprodukte eine Assemblierung des erfindungsgemäßen rekombinanten Virus-RNA *in trans* ermöglichen. Hierzu kann die Zelle z.B. weiterhin einen oder mehrere Vektoren enthalten, welche das N-Protein, das P-Protein oder/und das L-Protein *in trans* bereitstellen. Hierdurch wird eine Assemblierung von Nukleokapsiden des erfindungsgemäßen rekombinanten Virus in der Herstellungszelle ermöglicht.

Die Vermehrung des initial in der Virus-Herstellungszelle assemblierten rekombinanten Virus erfolgt in einer Virus-Vermehrungszelle, die mit dem erfindungsgemäßen Virus infiziert ist Zusätzlich enthält die Virus-Vermehrungszelle Helfersequenzen wie oben genannt, zur Bereitstellung des N-Proteins, oder/und des P-Proteins *in trans.* Vorzugsweise wird eine Virus-Vermehrungszelle verwendet, in der eine stabile Expression der Helfersequenzen, z.B. durch genomische Integration, erfolgt. Die Virus-Vermehrungszelle ist vorzugsweise eine Säugerzelle. Eine besonders bevorzugte Vermehrungszelle ist die von einer 293-Zelle, einer humanen embryonalen Nierenfibroblasten-Zelllinie abgeleitete Zelle H29 oder eine davon abgeleitete Zelle. Die Zelle H29 wurde am 05.11.2004 (DSM ACC 2702) gemäß den Bestimmungen des Budapester Vertrags bei der Deutschen Sammlung für Mikroorganismen und Zellkulturen GmbH, Braunschweig, Mascheroder Weg, hinterlegt. Weiterhin geeignet sind auch von Vero-Zellen, einer Nierenzelllinie aus der Afrikanischen Grünen Meerkatze, oder von LLCMK2-Zellen, einer Nierenzelllinie aus dem Rhesusaffen, abgeleitete Zellen, die stabil mit entsprechenden Helfersequenzen, z.B. SeV N- und P-Genen, transfiziert sind.

Ein weiterer Gegenstand der Erfindung ist daher eine Zelle, vorzugsweise eine eukaryontische Zelle und besonders bevorzugt eine Säugerzelle, die (i) ein DNA-Molekül, welches für das Genom des erfindungsgemäßen rekombinanten Virus oder/und die komplementäre Sequenz davon oder eine Vorstufe davon kodiert, oder/und (ii) ein RNA-Genom des erfindungsgemäßen Virus enthält. Die Zelle kann eine Vektor-Vermehrungszelle, eine Virus-Herstellungszelle oder eine Virus-Vermehrungszelle, wie zuvor erläutert, sein.

Wenn die Zelle eine Vektor-Vermehrungszelle, z.B. eine Plasmid-Vermehrungszelle, ist, kann jede beliebige zur Vermehrung des entsprechenden Vektors geeignete Zelle verwendet werden, z.B. auch eine prokaryontische Zelle wie etwa eine transformierte E. coli Zelle.

Wenn die Zelle eine Virus-Herstellungs- oder Vermehrungszelle ist, enthält sie Helfersequenzen zur Herstellung der Virusproteine N,P oder/und L *in trans.* Die in eine Virus-Herstellungszelle eingeführte DNA steht vorzugsweise unter Kontrolle eines heterologen Transkriptionssignals, wobei die Zelle günstigerweise weiterhin eine DNA enthält, die für eine heterologe DNA-abhängige RNA-Polymerase kodiert, welche das heterologe Transkriptionssignal erkennt und die Transkription der für das rekombinante Negativstrang RNA-Virus kodierenden DNA bewirkt.

Wenn die Zelle eine Virus-Vermehrungszelle ist, ist sie mit einem genomischen viralen RNA-Molekül, z.B. in Form eines Nukleokapsids, infiziert und enthält die Helfersequenzen in stabil exprimierbarer Form.

Noch ein weiterer Gegenstand der vorliegenden Erfindung ist ein Verfahren zur Herstellung eines erfindungsgemäßen rekombinanten Negativstrang RNA-Virus, umfassend die Schritte: (a) Bereitstellen einer Virus-Herstellungszelle, die mit einem DNA-Molekül transfiziert ist, das für das Genom eines Negativstrang RNA-Virus kodiert, enthaltend eine Mutation in dem Gen P, die zum Verlust der Genom-Replikationsfähigkeit ohne Verlust der Transkriptionsfähigkeit führt, und gegebenenfalls mindestens eine für ein heterologes Genprodukt kodierende Sequenz, und (b) Kultivieren der Wirtszelle unter Bedingungen, dass eine Transkription des DNA-Moleküls gemäß (a) erfolgt und das rekombinante Negativstrang RNA Virus initial gebildet wird. Die Wirtszelle ist vorzugsweise in der Lage, das N-Protein, das P-Protein und/oder das L-Protein *in trans* bereitzustellen, z.B. durch Transfektion mit den entsprechenden Helferplasmiden, die für die Proteine N, P oder/und L kodierende Sequenzen enthalten.

Zur Herstellung großer Mengen der Nukleokapside bzw. der Viruspartikel wird vorzugsweise eine Zelle verwendet, welche konstitutiv oder induzierbar die Proteine N,+ oder/und P, vorzugsweise zumindest das Protein P eines Negativstrang RNA-Virus stabil exprimiert. Die Erfindung betrifft somit auch ein Verfahren zur Vermehrung eines erfindungsgemäßen rekombinanten Negativstrang RNA-Virus, umfassend die Schritte: (a) Bereitstellen einer Virus-Vermehrungszelle, die mit dem Genom eines Negativstrang-RNA Virus infiziert ist, enthaltend eine Mutation in dem Gen P, die zum Verlust der Genom-Replikationsfähigkeit ohne Verlust der Transkriptionsfähigkeit führt, und gegebenenfalls mindestens eine für ein heterologes Genprodukt kodierende Sequenz, und (b) Kultivieren der Wirtszelle unter Bedingungen, dass eine Vermehrung des Virus erfolgt.

Noch ein weiterer Gegenstand der vorliegenden Erfindung ist eine pharmazeutische Zusammensetzung, die ein rekombinantes replikationsdefizientes und transkriptionskompetentes Negativstrang RNA-Virus, wie zuvor angegeben, oder dessen Nukleokapsid als Wirkstoff sowie gegebenenfalls als pharmazeutisch übliche Träger- oder/und Hilfsstoffe enthält. Die pharmazeutische Zusammensetzung ist für Anwendungen in der Human- und Veterinärmedizin geeignet. Sie kann insbesondere als Impfstoff oder für die Anti-Tumortherapie, insbesondere zur Anwendung bei Risikopatienten, wie etwa Kindern, älteren Personen oder/und Personen mit geschädigtem oder schwachem Immunsystem, verwendet werden. Die pharmazeutische Zusammensetzung kann dabei das Negativstrang RNA-Virus in seiner nativen viralen Hülle enthalten.

Besonders bevorzugt ist die Anwendung als Impfstoff, z.B. als Impfstoff gegen Pathogene, wie Viren, Bakterien oder Protozoen. Wenn das rekombinante Virus ein Transgen oder mehrere Transgene gleicher Herkunft, z.B. aus einem einzigen Pathogen, enthält, handelt es sich um eine monovalente Vakzine. Wenn das rekombinante Virus Transgene verschiedener Herkunft enthält, kann es auch als polyvalente Vakzine, z.B. als bivalente oder trivalente Vakzine, eingesetzt werden. Beispielsweise kann eine polyvalente Vakzine gegen mehrere pathogene Viren, z.B. gegen mehrere pathogene Negativstrang RNA-Viren, wie etwa humanes Parainfluenzavirus und RSV, erzeugt werden.

Ein erfindungsgemäßer Impfstoff ist in der Lage eine humorale Immunantwort, vorzugsweise die Bildung neutralisierender Antikörper, oder/und eine T-Zell-Immunantwort auszulösen. Besonders bevorzugt werden eine humorale Immunantwort und eine T-Zell-Immunantwort ausgelöst.

Die pharmazeutische Zusammensetzung kann in Form einer Lösung, Suspension, eines Lyophilisats oder in jeder anderen geeigneten Form vorliegen. Neben dem Wirkstoff kann die Zusammensetzung Mittel zur Einstellung des pH-Werts, Puffer, Mittel zur Einstellung der Tonizität, Netzmittel und dgl. sowie Adjuvanzien enthalten. Die Verabreichung kann auf üblichem Weg, z.B. oral, topisch, nasal, pulmonal etc. in Form von Aerosolen, Flüssigkeiten, Pulvern etc. erfolgen. Dabei wird eine therapeutisch wirksame Dosis des Virus an den Patienten verabreicht, wobei diese Dosis von der jeweiligen Anwendung (z B. Virotherapie oder Vakzine), von der Art der Erkrankung, dem Gesundheitszustand und Gewicht des Patienten, der Art der Verabreichung und der Formulierung etc. abhängt. Üblicherweise werden pro Applikation 10³ bis 10⁷ Viruspartikel, besonders bevorzugt etwa 10⁴ bis 10⁶ Viruspartikel verabreicht. Gegebenenfalls können mehrere unterschiedliche Viruspartikel gemeinsam verabreicht werden, z.B. im Fall von Kombinationsimpfungen. Die Verabreichung kann ein- der mehrfach, je nach Bedarf erfolgen.

Bevorzugte Anwendungsgebiete sind z.B. die Vorbeugung bzw. Behandlung von respiratorischen Viruserkrankungen

Weiterhin soll die Erfindung durch die nachfolgenden Abbildungen und Beispiele erläutert werden.

### Beispiele

### 1. Allgemeines

**Abbildung 1** zeigt die Morphologie eines Sendaivirus (SeV) nach Fields (Virology. Lippincott, Williams und Wilkins (2001), 4. Auflage; modifiziert). Das Genom besteht aus einer einzelsträngigen RNA, die mit den Proteinen N, P und L in Form eines Nukleokapsids vorliegt. Das Nukleokapsid ist von einer Membranhülle umgeben, in welche die Proteine HN und F (bestehend aus jeweils einer F₁- und F₂-Untereinheit) eingelagert sind. Mit der Innenseite der Membran ist das Protein M assoziiert, das gleichzeitig auch in Bindung mit den Nukleokapsidkomponenten vorliegt.

Das einzelsträngige negativ orientierte RNA-Genom des Sendaivirus-Wildtyp besteht aus 15384 Nukleotiden. Die Gene der 6 Strukturproteine liegen darauf in der Reihenfolge 3'-N-P/C-M-F-HN-L-5' vor **(****Abbildung 2****).** Zwischen den Genen liegen Übergänge aus 50-80 Nukleotiden, die jeweils eine stark konservierte Region von 22 Nukleotiden enthalten: das Terminationssignal des vorangegangenen Gens, eine intergenische Sequenz und das Startsignal für das folgende Gen. Eine Einheit aus Startsignal, offenem Leserahmen (ORF), gegebenenfalls nicht-translatierten Regionen und Terminationssignal wird als Transkriptionskassette bezeichnet. Vor dem N-Gen befindet sich eine 55 Nukleotide lange Leader-Sequenz (Id), die transkribiert, allerdings nicht translatiert wird. Hinter dem L-Gen befindet sich die 54 Nukleotide lange Trailersequenz (tr). Die ld- und tr-Region fungieren als genomische und antigenomische Promotoren für die Replikation von Genom bzw. Antigenom. Die durch Transkription gebildeten mRNA-Moleküle sind mit Ausnahme der P/C-RNA monocistronisch.

Der Vermehrungszyklus des Sendaivirus umfasst den Eintritt in die Wirtszelle, die Transkription und Translation sowie die Replikation und Virusreifung mit anschließender Ausschleusung neu produzierter Viren. Am Vorgang der Transkription sind insbesondere die Proteine N, P und L beteiligt, wobei L die virale RNA-abhängige RNA-Polymerase mit allen katalytischen Aktivitäten darstellt. Da das Genom des Sendaivirus in Negativstrang-Orientierung vorliegt, kann die virale RNA nicht direkt in Proteine übersetzt werden. Zunächst erfolgt eine primäre Transkription zu mRNAs durch RNA-Polymerase, die mit dem Nukleokapsid assoziiert in die Zelle gebracht wird.

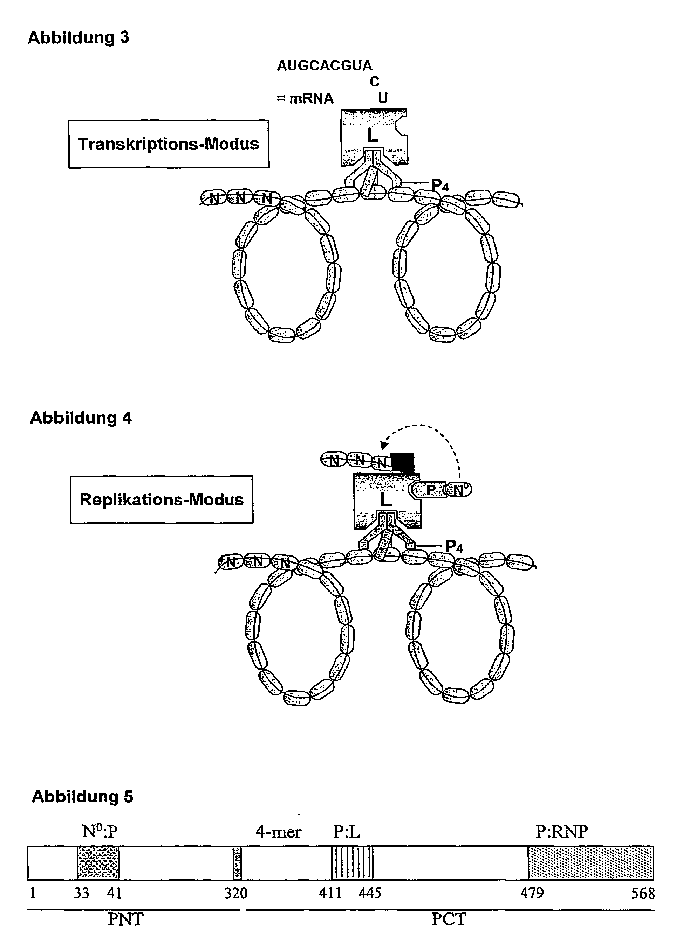

**Abbildung 3** zeigt eine schematische Darstellung des Transkriptionsmodus des Sendaivirus. Dabei wandert der Polymerase-Komplex, bestehend aus einem L-Protein und einem Homotetramer aus P-Proteinen, auf der mit N-Proteinen verpackten RNA in Richtung des 5'-Endes entlang. Dabei wird die genetische Information auf der genomischen Negativstrang RNA abgelesen und in Positivstrang mRNA transkribiert.

Die Replikation des Genoms umfasst die Herstellung neuer Virusgenome mit negativer Polarität. Hierzu werden zunächst Antigenome gebildet, die dann als Matrizen für die Bildung der Genome dienen. Da die Transkription am 3'-Ende des Genoms (Id) beginnt, ist eine Umschaltung vom Transkriptions- in den Replikationsmodus erforderlich. Dieses Umschalten wird von der Menge an freiem N-Protein in der Zelle bestimmt. Erst wenn ausreichend N-Protein nach der Translation von mRNA-Molekülen gebildet worden ist, kann eine Replikation erfolgen. Nachdem ein Antigenom, das über seine ganze Länge mit N-Proteinen komplexiert ist, vorliegt, kann dieses als Matrize zur Herstellung von weiteren Genomen dienen. Diese werden ebenfalls direkt mit N-Proteinen verpackt. Für den Prozess der Replikation sind wiederum die Proteine N, P und L verantwortlich **(****Abbildung 4****).**

Während der Virusreplikation werden durch die steigende Anzahl an mRNA-Molekülen auch zunehmend virale Proteine synthetisiert. Dann werden Komplexe aus viraler RNA und viralen Proteinen (Nukleokapside) in Form von sekretorischen Vesikeln zur Zytoplasmamembran transportiert, wo es zur Umhüllung mit viralen Oberflächenproteinen und Knospung von Viruspartikeln kommt.

Im Rahmen der vorliegenden Erfindung werden rekombinante Virusmutanten bereitgestellt, bei denen die Funktionen der Transkription und Replikation entkoppelt sind, d.h. die Viren sind transkriptionskompetent, aber replikationsdefizient. Die fehlende Genomreplikationsfunktion muss für die Herstellung von Viruspartikeln bzw. deren Nukleokapsiden durch Helferzellen kompensiert werden, die das fehlende oder funktionsdefiziente virale Protein *in trans* komplementieren. Eine bevorzugte solche Helferzelle ist die Zelllinie H29 (Willenbrink und Neubert, J. Virol. 68 (1994), 8413-8417). Diese Zelllinie wurde im Rahmen der vorliegenden Anmeldung gemäß den Bestimmungen des Budapester Vertrags unter dem Aktenzeichen DSM ACC2702 am 05.11.2004 hinterlegt. Zur Herstellung von replikationsdefizienten, aber transkriptionskompetenten Viren wurde das für das P-Protein kodierende Gen nicht vollständig entfernt, sondern nur eine für die Genomreplikation essentielle Domäne. So war aus früheren Arbeiten (Curran, Virology 221 (1996), 130-140; Curran et al. J. Virol. 69 (1995), 849-855) bekannt, dass in einem *in vitro* System bei Deletion der Aminosäuren 2-77 des Proteins P die Genomreplikation inhibiert wird, während die virale Transkription aktiv bleibt.

**Abbildung 5** zeigt eine schematische Darstellung des P-Proteins mit seinen N- und C-terminalen Domänen (PNT, PCT), der Tetramerisierungsdomäne (Aminosäuren 320-446), der P:L-Domäne (Aminosäuren 411-445) und der P:RNP-Bindedomäne (Aminosäuren 479-568). Für eine Abschaltung der viralen Genomreplikationsfunktion bei gleichzeitiger Beibehaltung der Fähigkeit zur viralen mRNA-Synthese wurde eine Deletion der ersten 77 Aminosäuren des Proteins P ausgewählt. Dabei wurde erstmals eine entsprechende Sendaivirus-Mutante (SeV-PΔ2-77) erzeugt, bei der der 5'-terminale Bereich des P-ORF deletiert wurde. Von diesen Viren können nur N-terminal verkürzte P-Proteine kodiert werden. Infektionsstudien zeigten, dass die Virusmutante in Zellkultur nicht vermehrungsfähig ist. Mit Hilfe der Helferzelllinie H29 (DSM ACC2702), die unter anderem das erforderliche Wildtyp-P-Protein bereitstellt, kann eine Vermehrung der Virusmutante erreicht werden. Die Effizienz der Virusvermehrung beträgt ca. 45 % im Vergleich zu Wildtyp-Sendaiviren.

Nach Infektion ist die erfindungsgemäße Virusmutante in der Lage, viruskodierte Transgene in infizierten Zellen zu exprimieren. Das von der Virusmutante bereitgestellte verkürzte Protein P unterstützt ausreichend die sekundäre virale mRNA-Synthese. Die Synthese viruskodierter Proteine erstreckt sich in den infizierten Zellen über mehrere Tage und ist nur um den Faktor von ca. 10 gegenüber dem Viruswildtyp reduziert, so dass beim Einsatz der Mutante als Vakzine eine ausreichende Immunantwort sicher erwartet werden kann.

### 2. Herstellung von Basiskonstrukten für replikationsdefiziente Sendaivirus-Vektoren (SeVV)

### 2.1 Herstellung eines cDNA-Konstrukts pSeV-X

Es wurde eine kodierende Transkriptionskassette in den 3'-Bereich von SeV, Stamm Fushimi (ATCC VR105), eingefügt. Bei allen Manipulationen des Genoms muss unbedingt beachtet werden, dass die Gesamtzahl der Nukleotide des rekombinanten SeV Genoms durch sechs teilbar ist ("*rule of six*")*.*

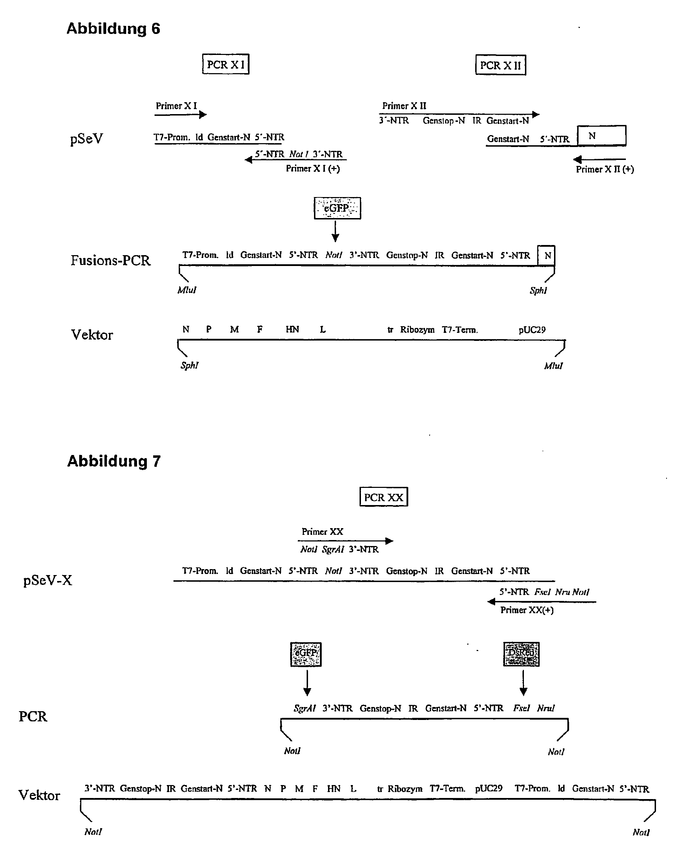

Ausgehend von der als Matrize verwendeten cDNA pSeV wurden zwei PCR-Fragmente PCR X I und PCR X II für die Herstellung von pSeV-X bereitgestellt **(****Abbildung 6****).**

PCR X I (370 bp) besteht aus der Sequenz des T7-Promotors (T7-Prom.), der leader (Id)-Sequenz, dem N-Genstart mit 5'-NTR bis vor das StartCodon des N-ORF (Open Reading Frame). Über den reverse-Primer X I (+) **(Tabelle 3)** wurden eine singuläre *Notl*-Restriktionsschnittstelle und 24 Nukleotide der N-Genstoppsequenz angefügt. Die 24 Nukleotide der N-Genstoppsequenz von PCR XI, eingefügt durch den mutagenen Primer XI (+), dienen im darauf folgenden Fusionsschritt als der mit PCR XII überlappende Bereich.

PCR XII (970 bp) besteht aus der Sequenz des N-Genstarts und des ersten Drittel des N-ORF. Über den *forward-*Primer XII wurde die Sequenz der 3'-NTR N und die Genstopp-Sequenz von N, wie auch die intergene Region (IR) angefügt. Der reverse Primer XII (+) bindet im ersten Drittel des N-ORF kurz hinter der im SeV Genom singulären *Sphl* Schnittstelle. Das Amplikon PCR XI war im 3'-Bereich komplementär zum 5'-Bereich von PCR X II. Durch diesen überlappenden Bereich konnten die beiden PCR-Fragmente X I und XII fusioniert werden. Nach erfolgter PCR konnte das Fusions-Produkt (1310 bp) durch Restriktionsspaltung mit den Enzymen *Mlul* und *Sphl* in den ebenfalls mit *Mlul* und *Sph*/ behandelten Vektor pSeV eingefügt werden. Aus den erhaltenen Klonen wurde durch Plasmid-Präparation Plasmid-DNA isoliert und durch Restriktionsanalyse und Sequenzierung auf die korrekte Insertion der Transkriptionskassette überprüft. Es stand somit das cDNA Konstrukt pSeV-X zur Verfügung.

Um die Erzeugung von rekombinanten Viren leicht verfolgen zu können, wurde nun das Gen für das *enhanced green fluorescent protein* (eGFP) in die leere Kassette von pSeV-X eingefügt. Der eGFP-ORF wurde durch PCR aus dem Expressionsplasmid pEGFP-N1 (Fa. Clontech) amplifiziert, wobei durch mutagene Primer die Einhaltung der *"rule of six"* und das Anfügen von zwei flankierenden *Notl*-Schnittstellen erreicht wurde. Das entstandene 771 bp große PCR-Fragment wurde mit dem Restriktionsenzym *Notl* gespalten und ein 738 bp großes Fragment durch Gelelution isoliert, das über die Notl-Schnittstelle von pSeV-X in dessen "leere" Transkriptionskassette pSeV-X eingesetzt wurde. Nach Transformation von *E.coli*, Plasmid-Präparation und anschließender Sequenzierung des über PCR eingefügten eGFP-Leserahmens stand das cDNA Konstrukt pSeV-eGFP zur Verfügung.

### 2.2 Herstellung des cDNA-Konstrukts pSeV-X-X

Durch das Konstrukt pSeV-X-X sollten zwei zusätzliche Transkriptionskassetten zur Verfügung gestellt werden, in welche zwei Transgene eingebaut werden können. Die Verwendung von pSeV-X-X als Basisvektor für die Herstellung der replikationsdefizienten Vektoren sollte es ermöglichen, den Vektor mit multivalenten, z.B. trivalenten, Eigenschaften auszustatten.

pSeV-X-X wurde über eine PCR-Reaktion hergestellt, bei der pSeV-X als Template diente **(****Abbildung 7****).** Der Primer XX-*forward* hybridisiert mit pSeV-X im Bereich der *Notl*-Schnittstelle und dem 3'-NTR der zu integrierenden, zweiten Transkriptionskassette. Eine singuläre *SgrAl-*Restriktionsschnittstelle wurde mit Hilfe des XX- *forward* Primers zwischen der Notl-Schnittstelle und dem 3'-NTR eingeführt. Sie dient als singuläre Restriktionsschnittstelle für die spätere Insertion des ORFs eines Transgens. Im PCR-Produkt XX folgen Genstopp, intergene Region (IR), Genstart und 5'-NTR. Durch den Primer XX (+), der mit dem 5'-NTR hybridisiert, wurden die singulären Restriktionschnittstellen *Fsel* und *Nru l* eingefügt. Die Fsel-Schnittstelle dient dem Einbau des ORFs eines zweiten Transgens. Die singuläre *Nru l*-Schnittstelle wurde vorausschauend einkloniert, um bei Bedarf eine dritte Transkriptionskassette integrieren zu können. Der Primer XX (+) hybridisiert im 3'-Bereich mit der Sequenz der *Notl*-Schnittstelle von pSeV-X. Das PCR-Produkt XX (220 bp) wurde mit dem Restriktionsenzym *Notl* behandelt und ein Fragment von 144 bp durch Gelextraktion isoliert. Dieses Fragment, konzipiert unter Einhaltung der *"rule of six",* konnte dann in das ebenfalls mit *Notl*-behandelte Plasmid pSeV-X eingebaut werden. Nach Überprüfung der korrekten Orientierung des *Notl* PCR-Fragments XX und Verifizierung der Sequenz stand das Plasmid pSeV-X-X bereit. In die singulären Schnittstellen *SgrAl* und *Fsel* können beliebige Transgene integriert werden.

Für die Untersuchungen in dieser Arbeit wurden die beiden Transkriptionskassetten (X) von pSeV-X-X mit Leserahmen von zwei verschiedenen fluoreszierenden Proteinen versehen. Zum einen wurde der Leserahmen für das fluoreszierende Protein eGFP aus dem Expressionsplasmid pEGFP-N1 durch PCR-Reaktion unter Einhaltung der "*rule of six*" amplifiziert, wobei mit Hilfe mutagener Primer zwei flankierende SgrAl-Schnittstellen angefügt wurden. Nach Restriktionsspaltung mit *SgrAl* und Gelelution konnte das etwa 738 bp große Fragment in die erste Transkriptionskassette von pSeV-X-X eingebaut werden (pSeV-eGFP-X). Auf gleiche Weise wurde zum anderen der ORF des fluoreszierenden Proteins DsRed (aus Plasmid "pDsRed", Fa. Clontech) unter Einhaltung der *"rule* of *six"* über PCR-Reaktion mit den Restriktionsschnittstellen von *Fsel* versehen, die DNA gespalten, geleluiert und dieses Fragment (702 bp) anschließend in die zweite Transkriptionskassette im 3'-Bereich von pSeV-eGFP-X einkloniert. Es entstand das genomische SeV cDNA Konstrukt pSeV-eGFP-DsRed.

### 3. Herstellung replikationsdefizienter Sendaivirus-Vektoren (SeVV)

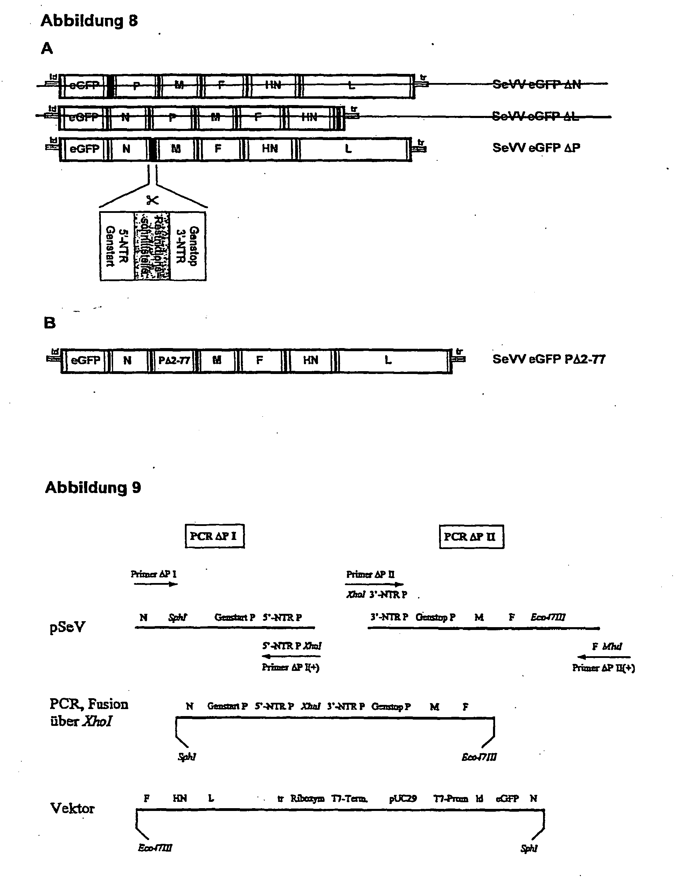

Es wurden cDNA Konstrukte pSeVV-eGFP-ΔP hergestellt, die für replikationsdefiziente Sendaiviren kodieren, in denen das Gen für das Protein P deletiert ist Hierzu musste unter Einhaltung der *rule of six* ein Leserahmen das Gens P deletiert werden, wobei eine nicht-kodierende Translaiptionskassette an der entsprechenden Position erhalten bleiben sollte **(****Abbildung 8A****).**

Durch Einbau einer Restriktionsschisttstelle anstelle des deletierten ORF sollte in dem cDNA Konstrukt pSeVV-eGFP -ΔP für spätere Anwendungen eine weitere funktionelle Transkriptionskassette zur Verfügung stehen, in die bei Bedarf ein weiteres Transgen eingesetzt werden kann.

Als weitere Variante eines replikationsdefizienten SeVV wurde die Deletionsmutante pSeVV-eGFP-PΔ2-77 hergestellt, die für ein N-terminal verkürztes P Protein kodiert, dem die Aminosäuren 2 bis 77 fehlen **(****Abbildung 8B****).**

Die Klonierung von pSeVV-eGFP-ΔP soll im folgenden Absatz ausführlich beschrieben werden.

### 3.1 Klonierung der cDNA-Konstrukte pSeVV-eGFP-ΔP und pSeVV-eGFP-PΔ2-77

Der ORF des P-Proteins wurde aus dem cDNA Konstrukt des replikationskompetenten Virus pSeV-eGFP entfernt, um die neue cDNA pSeVV-eGFP-ΔP, kodierend für den replikationsdefizienten Vektor, herzustellen. Anstatt des P-ORF wurde eine *XhoI*-Restriktionsschnittstelle eingesetzt.

Für die Klonierung von pSeVV-eGFP-ΔP wurden zwei PCR-Fragmente namens PCR ΔP I und PCR ΔP II hergestellt und anschließend fusioniert. Als Template für die beiden PCR-Reaktionen diente pSeV-eGFP. Beim Fragment PCR ΔP I (1272 bp) wurde über den *forward-*Primer ΔP I (= N-578; **Tabelle 3)** eine Hybridisierung mit dem Template im Bereich des N ORF vor einer singulären *Sphl*-Stelle erreicht. Der *reverse*-Primer ΔP I (+) hybridisiert mit dem Template im 5'-NTR Bereich des P-Gens bis vor das ATG-Codon von P und fügt dort die Restriktionsschnittstelle *XhoI* ein.

Das Fragment PCR ΔP II besteht aus 1938 bp, als Template dient auch hier pSeV. Der *forward*-Primer ΔP II hybridisiert mit einem Teil der 5'-NTR P Sequenz und hängt eine *Xhol*-Schnittstelle an. Der *reverse*-Primer von PCR ΔP II (+) bindet im ORF des F Gens hinter einer singulären *Eco*47III Stelle und weist zusätzlich eine artifizielle *Mlul*-Stelle auf.

Beide PCR-Fragmente ΔPI und ΔP II wurden über die Xhol-Stelle kombiniert. Das Fusionsprodukt - bestehend aus einer Teilsequenz des N ORF, der nicht-kodierenden P-Transkriptionskassette mit eingefügter *Xho*I-Restriktionsschnittstelle, dem M sowie einem Viertel des F ORF - wurde mit den Restriktionsenzymen *Sphl* und *Miu*l gespalten, zwischenkloniert und sequenzverifiziert. Aus einem Subklon mit korrekter Sequenz wurde ein 3006 bp großes *Sph*I-*Eco*47III Fragment geschnitten, welches in den identisch behandelten Vektor pSeV-eGFP ligiert wurde. Ein entsprechender pSeVV-eGFP-ΔP Klon (genomische virale cDNA) stand nach Sequenzüberprüfung nun für die Herstellung des replikationsdefizienten SeVV-eGFP-ΔP bereit **(****Abbildung 9****).**

Zur Konstruktion der Deletionsmutante pSeVV-eGFP-PO2-77 wurde eine PCR mit zwei mutagenen Primem angewendet Der *forward* Primer "*XhoI* PA2-77" enthält eine *Xho*I-Stelle, gefolgt von einem ATG-Startcodon sowie Codons für die Aminosäuren 78 bis 86 des P Proteins. Der reverse Primer "PΔ2-77 (+) *XhoIl*" enthält die letzten 10 Codons des P Proteins und eine *Xho*I-Stelle. Der Leserahmen des N-terminal um 76 Aminosäuren verkürzten P-Proteins wurde mittels PCR, ausgehend vom Template pSeV, unter Einhaltung der *rule of six* hergestellt. Das Xhol-gespaltene, 1488 bp große Fragment wurde über zwei Klonierungsschritte in die nicht-kodierende Transkriptionskassette von pSeVV-eGFP-ΔP an der Position des ursprünglichen P-ORF eingefügt Nach Sequenzüberprüfung stand nun auch ein genomischer cDNA Klon für die Herstellung des replikationsdefizienten SeVV-eGFP-PΔ2-77 bereit.

Die Deletion der Codons 2 bis 77 im P ORF hat zur Folge, dass im Falle der Nichtstrukturproteine auch das V und W Protein N-terminal verkürzt sind und von der C-Familie nur noch C' - ebenfalls trunkiert - kodiert bleibt; die Proteine C, Y1 und Y2 können aufgrund fehlender Startcodons nicht mehr von der verkürzten mRNA translatiert werden.

Die Sequenzen der Primer zur Klonierung von pSeVV-eGFP-ΔP sind unter Aufstellung der verwendeten DNA-Oligonukleotide aufgelistet **(Tabelle 3)**. Die hergestellten PCR-Produkte PCR I und PCR II wurden fusioniert und mit Hilfe des forward-Primers von PCR I und des reverse-Primers von PCR II amplifiziert. Anschließend wurden die Fusions-PCR-Produkte mit Restriktionsenzymen, die in pSeV-eGFP singulär vorkommen und die Insertion des entsprechenden Fusionsproduktes in pSeV-eGFP zulassen, gespalten (z.B.: *Narl* bei Klonierung pSeVV-eGFP-ΔN, siehe Tabelle 1). Das gereinigte Spaltprodukt wurde durch Ligation in den ebenfalls mit den entsprechenden Enzymen verdauten Vektor pSeV-eGFP eingefügt

**Tabelle 1: Übersicht über die bei der Klonierung von pSeVV-eGFP-ΔP-verwendeten Primer und Restriktionsschnittstellen**

| | pSeVV-eGFP-ΔP |
|---|---|
| Primerpaar PCR I | ΔpI, ΔP I (+) |
| Primerpaar PCR II | ΔP II. ΔP II (+) |
| **RSS¹ der Transkriptions-kassette** | *Xhol* |
| **RSS⁵ der Klonierung** | *SphI* + *Eco47III* |

| | |
|---|---|
| § RSS = Restriktionsschnittstelle | |

*E.coli* Zellen wurden mit einem Teil der Ligationsansätze transformiert und Plasmid-DNA der erhaltenen Klone durch Plasmid-Mini-Präparation isoliert. Nach Überprüfung der korrekten Sequenz durch Restriktionsanalyse und Sequenzierung wurde von einem positiven Klon eine Plasmid-Präparation angefertigt (DNA Maxi Prep-Kit, Qiagen), und so stand pSeVV-eGFP-ΔP für die Herstellung von rekombinanten Deletionsmutanten bereit.

### 4. Herstellung von replikationsdefizienten Virusmutanten

Aus dem cDNA Konstrukte pSeVV-eGFP-ΔP wurden in Zellkultur replikationsdefiziente SeV-Vektoren (SeVV-eGFP-ΔX) hergestellt

### 4.1 Initiale Herstellung von SeVV-eGFP-ΔP

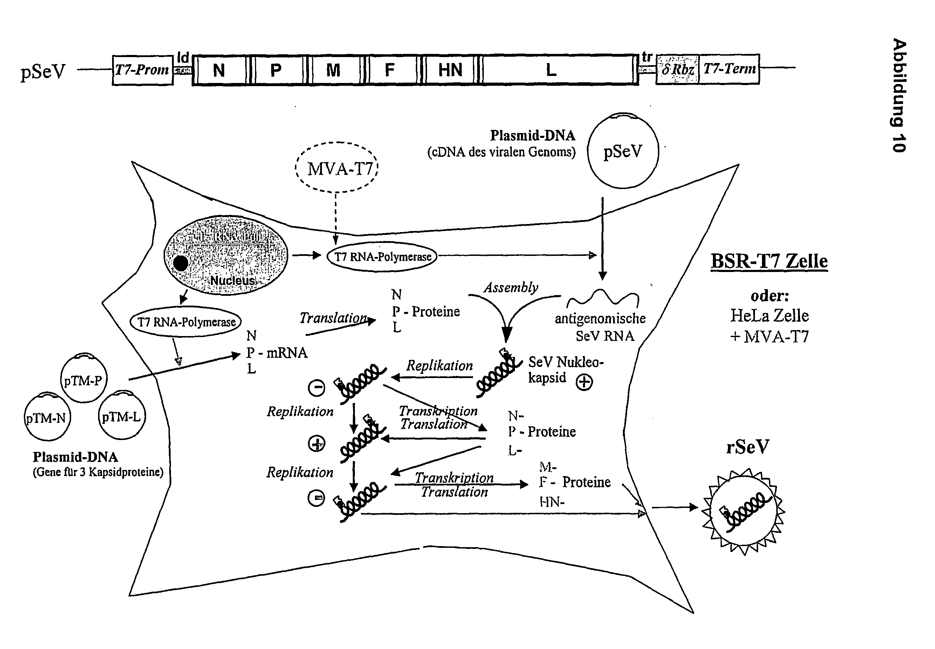

Für die Herstellung von rekombinanten SeV mit vollständigem Genom werden
- entweder die Zelllinie "BSR-T7", die die T7 RNA-Polymerase stabil exprimiert (Buchholz et al: (1999) J. Virol. 73, 251-259)
- oder Zellkulturen mit dem rekombinanten Vaccinia-Virus MVA-T7, das die T7 RNA-Polymerase exprimiert (Sutter et al. (1995) FEBS 371, 9-12) infiziert, und
- mit der cDNA des viralen Genoms (pSeV) sowie den Plasmid-kodierten Genen N, P und L transfiziert (pTM-N, -P, -L; Elroy-Stein et al. (1989) PNAS 86, 6126-6130). Die T7-Polymerase transkribiert nun das virale Antigenom oder/und die komplementäre Sequenz und die Gene N, P und L Das über pTM-N exprimierte N-Protein verpackt die synthetisierte virale antigenomische oder/und komplementäre DNA, und dieses Nukleokapsid-Core (RNP) bildet zusammen mit den Proteinen P und L den Replikationskomplex über den wiederum genomische RNA hergestellt und zu Nukleokapsiden verpackt werden kann (Leyrer et al., J. Virol. Meth. 75 (1998), 47-55). Im Anschluss daran erfolgt die Transkription aller viruskodierter Proteine und Replikation weiterer viraler Genome **(****Abbildung 10****).**

Im Unterschied zur beschriebenen Herstellung von rekombinanten SeV wt mit vollständigem Genom fehlt in der Plasmid-kodierten cDNA von pSeVV-eGFP-ΔP die genetische Information für das Gen P. Demzufolge sind die initial hergestellten Nukleokapside nur in der Lage, die Gene N, und L zu exprimieren. Die zur Vermehrung von SeVV-eGFP, -ΔP Nukleokapsiden benötigte Menge des fehlenden Proteins muss daher ausschließlich über die T7-Promotor-gesteuerte Expression das Plasmid-kodierten Gens P zur Verfügung gestellt werden.

Die Herstellung das replikationsdefizienten SeVV-eGFP -ΔP erfolgte analog zur Herstellung von replikationskompetenten SeV Varianten in HeLa-Zellen (ATCC CCL2) oder BSR-T7-Zellen. Nach 48-stündiger Inkubation der HeLa-Zellen wurde untersucht, ob virale Partikel von SeVV-eGFP -ΔP in die Kulturüberstände abgegeben worden waren und wie viele initiale Detetionsmutanten entstanden sind.

### 4.2 Detektion initial hergestellter SeVV-eGFP -ΔP

Die Überstände von HeLa-Zellen oder BSR-T7-Zellen, in denen initial SeVV-eGFP -ΔP hergestellt worden sein sollten, wurden auf die Anwesenheit dieser viralen Vektoren untersucht SeVV-eGFP -ΔP hat im Gegensatz zum rekombinanten SeV wt, im 3'-Bereich das Reportergen für eGFP integriert. Dieser Detektionsmarker wurde nun verwendet, um zu analysieren, wie viele SeVV-eGFP -ΔP gebildet worden waren.

In parallelen Ansätzen wurden 5x10⁵ Verozellen (ATCC CCL18) mit je 1 ml Zellkultur-Überstand der bei der initialen Herstellung von SeVV-eGFP -ΔP transfizierten HeLa-Zellen und zeitgleich mit SeV wt (MOI = 3) zur Transkomplementation des fehlenden Proteins co-infiziert Als Kontrolle wurden Verozellen entweder nur mit 1 ml initial hergestellten SeV-eGFP oder aber mit initial hergestellten SeV-eGFP und zeitgleich mit SeV wt (MOI = 3) infiziert.

Das Ergebnis der Koinfektion von Zellen mit Kultur-Überständen der Herstellung von SeVV-eGFP -ΔP und SeV wt zeigt, dass die Virusmutante SeVV-eGFP-ΔP initial herstellbar ist und nach initialer Herstellung auch in der Lage ist, Zellen zu infizieren, was sich durch eine nachweisbare eGFP-Transgenexpression detektieren lässt Es können initial etwa 13x10² SeV-eGFP, 3,2 x 10² SeVV-eGFP-ΔP Virusmutanten hergestellt werden.

### 5. Vermehrung von SeVV-eGFP-ΔP

Es wurde untersucht, ob die Vektoren SeVV-eGFP-ΔP vermehrbar, das heißt biologisch funktionell sind. Die Replikationsfähigkeit sollte zunächst durch Bereitstellung der Proteine des fehlenden Gens P durch virale Transkomplementation mit SeV untersucht werden.

### 5.1 Nachwels der Vermehrbarkeit von SeVV-eGFP-ΔX

Zunächst musste nachgewiesen werden, dass die Mutanten bei entsprechende Transkomplementation durch das SeV wt Virus in der lage sind sich zu vermehren und dabei umliegende Zellen infizieren. Für die Untersuchung der Vermehrbarkeit von SeVV-eGFP-ΔP bietet sich wiederum die Koinfektion von Zellen mit SeVV-eGFP-ΔP und SeV wt an. Die Zellen wurden in diesem Versuch mit SeV wt in niedriger MOI infiziert, mit SeVV-eGFP-ΔP coinfiziert und über mehrere Tage inkubiert, um so die Ausbreitung der SeVV-eGFP-ΔP Vektoren durch die zunehmende Anzahl fluoreszierender Zellen detektieren zu können.

5 x 10⁵ Verozellen wurden zeitgleich mit durchschnittlich 100 initial hergestellten SeVV-eGFP -ΔP und SeV wt (MOI = 0,2) infiziert Als Positivkontrolle diente die Koinfektion von Verozellen mit etwa 100 Partikeln des replikationskompetenten Virus SeV-eGFP und SeV wt Während einer 48-stündigen Inkubationsphase konnte eine Vermehrung aller drei Deletionsmutanten beobachtet werden: Zunächst fluoreszierten jeweils nur einzelne Verozellen, die mit SeVV-eGFP-ΔP und SeV wt koinfiziert worden waren. Nach etwa 24 h wurden aus diesen Zellen neu synthetisierte Viruspartikel entlassen, die nun in der Lage waren, in nebenliegende Zeiten einzudringen. Wurden diese Zeiten wiederum zeitgleich mit SeVV-eGFP-ΔP und SeV wt infiziert, kam es ebenfalls in solchen Zellen zu einer detektierbaren Fluoreszenz-Erscheinung. Die Vermehrung von SeW-eGFP-ΔP, in mit wt koinfizierten Zellen konnte durch diese "Schweifbildung" fluoreszierender Zellen 48 h p.i. und darüber hinaus detektiert werden.

Durch diesen Versuch wurde sichergestellt, dass die genomischen cDNA-Konstrukte, aus denen SeVV-eGFP-ΔP, entstanden ist, in allen Bereichen funktionell sind. Weiterhin konnte gezeigt werden, dass eine Vermehrung der Virusmutanten bei Zugabe des fehlenden viralen Proteins möglich ist Das ausschließlich von wt Virus bereitgestellte Protein (zB. P) ist ausreichend, um beide, die Deletionsmutante und den wt, zu vermehren, das heißt auch eine möglicherweise suboptimale Menge an P-Protein führt zur Bildung funktioneller Nukleokapside von SeV wt und SeVV-eGFP-ΔP. Durch die Bereitstellung des fehlenden Proteins durch den Transkomplementationspartner SeV wt konnte eine sichtbare Vermehrung von SeVV-eGFP-ΔP, gemessen durch die Zunahme fluoreszierender Zellen in den Kulturansätzen, erreicht werden.

### 5.2 Bestimmung der erforderlichen Proteine zur Vermehrung von SeVV-eGFP-ΔP

Als nächstes wurde untersucht, welche Proteine im Fall der Vermehrung von SeVV-eGFP-ΔP durch eine Helferzelle zur Verfugung gestellt werden müssen, sollten die SeV Proteine N, P und L unabhängig voneinander synthetisiert werden können. Für eine unabhängige Synthese der SeV Proteine N, P und L sanden die drei rekombinanten Vaccinia Viren (VV) W-N, W-P, W-L zur Verfügung, welche die SeV Proteine N, P oder L rekombinant exprimieren (Graef, H. (1994) Funktionelle Charakterisierung des rekombinanten Sendai-Virus Large (L) Proteins. Dissertation, Eberhard-Karis-Universität Tübingen).

Es wurden 1 x 10⁶ Verozellen simultan mit SeVV-eGFP-ΔP oder SeV-eGFP (MOI = 0,01) und VV co-infiziert. W-N, -P und -L wurden dabei einzeln oder in Kombinationen eingesetzt (MOI = 0,5). Nach einer einstündigen Adsorptionszeit erfolgte ein Mediumwechsel mit DMEM + 10 % fötales Kälberserum (FKS) + Cytosin-Arabinosid (AraC) (100 µg/ ml) und die Zellen wurden für 72 h bei 33 °C inkubiert, wobei das Medium täglich gewechselt wurde, um neues AraC zuzugeben.

Die Ausbreitung von SeVV-eGFP-ΔP wurde über die eGFP-Expression nach 72 h analysiert In dieser Zeit ergab sich in den positiven Ansätzen eine Vermehrung grün fluoreszierender Zellen von einer Initialzelle zu 10-30 benachbarten, fluoreszierenden Zellen.

Die Ergebnisse der Untersuchung, welche SeV Proteine notwendig sind, um SeVV-eGFP-ΔP in Verozellen zu vermehren, sind in **Tabelle 2** zusammengefasst. SeVV-eGFP-ΔP kann allein durch die Expression von SeV P-Proteinen mit Hilfe von VV-P in Verozellen vermehrt werden.

**Tabelle 2: Zur Vermehrung von SeVV-eGFP-ΔP benötigte SeV Proteine**

| | *in vitro*-Vermehrung mittels VV | |
|---|---|---|
| SeW-eGFP-ΔP | P + | (N + P) + |

Zur Vermehrung von SeVV-eGFP-ΔP reicht die Expression von SeV P-Protein in der Helferzelle aus.

### 5.3 Unterstützung der Amplifikation von SeVV-eGFP-ΔP durch H29 Helferzellen

Für die Untersuchungen zur Vermehrung von SeVV-eGFP-ΔP mit Hilfe der H29 Zelllinie wurden 1 x 10⁶ H29 Zellen in vier verschiedenen Ansätzen mit ca. 100 initial in HeLa-Zellen hergestellten -ΔP Viruspartikeln bzw. als Kontrolle für die erfolgreiche Vermehrung replikationsfähiger SeV in H29 Zellen mit etwa 100 SeV-eGFP Viruspartikeln infiziert Im Zeitraum von 1 bis 10 d p.i. erfolgte die Untersuchung auf Vermehrung von SeVV-eGFP-ΔP durch Nachweis einer schweifartigen Ausbreitung der fluoreszierenden Zellen (Spotbildung), ausgehend von einer initial infizierten H29 Zelle.

Im Kontrollansatz konnte eine Vermehrung von SeV-eGFP, ausgehend von singulär fluoreszierenden Zellen 1 d p.i. zu Spots mit bis zu 50 fluoreszierenden Zellen 3 d p.i. beobachtet werden. Damit war sichergestellt, dass es durch den gewählten Versuchsaufbau zu einer Vermehrung von SeV kommt.

Im Ansatz mit SeW-eGFP-ΔP konnten 1 d p.i. an die hundert initial infizierte Einzelzellen detektiert werden. 3 d p.i. hatten sich etwa 70 % der fluoreszierenden Einzelzellen zu Spots, mit bis zu 30 fluoreszierenden Zellen, entwickelt. Damit konnte eindeutig eine Verbreitung von SeVV-eGFP-ΔP auf umliegende H29 Zellen beobachtet werden. Neben H29 (einem Derivat von humanen 293- Nierenzellen) sind auch Derivate von Vero-Zellen (Nierenzellen der Afrikanischen Grünen Meerkatze) und Derivate von LLCMK2-Zellen (Nierenzellen des Rhesusaffen), stabil transfiziert mit SeV P-Gen, oder mit SeV P- und N-Genen, zur Virusvermehrung geeignet. Somit ist es erstmals möglich, einen viralen SeV Vektor, dessen P-ORF deletiert ist, zu vermehren. Auf die Charakterisierung der Vermehrung von SeVV-eGFP-ΔP soll im folgenden Unterpunkt eingegangen werden.

### 5.4 Vermehrung von SeVV-eGFP-ΔP auf H29-Zellen

SeVV-eGFP-ΔP kann durch die von H29 Helferzellen produzierten SeV P-Proteine amplifiziert werden. Die freigesetzten P-Deletionsmutanten sind in der Lage, umliegende H29 Zellen zu infizieren. Die Ausbreitung von SeVV-eGFP-ΔP sorte nun im Vergleich zu der Ausbreitung des replikationskompetenten SeV-eGFP analysiert werden.

Hierzu wurden 1 x 10⁶ H29 Zellen mit durchschnittlich 100 SeVV-eGFP-ΔP oder SeV-eGFP infiziert. 3, 5 und 10 Tage p.i. erfolgte die Detektion von grün fluoreszierenden Zellen mit Hilfe des Fluoreszenzmikroskopes.

Eine Vermehrung von SeVV-eGFP-ΔP konnte erfolgreich durch die zelluläre Bereitstellung von SeV P-Proteinen durchgeführt werden. Zu allen untersuchten Zeitpunkten war erkennbar, dass sich SeVV-eGFP-ΔP und SeV-eGFP auf H29 Zellen effizient vermehren.

Im Gegensatz dazu konnte eine Ausbreitung von SeVV-eGFP-ΔP auf Zellen, die das fehlende P Protein nicht zur Verfügung stellen ("Zielzellen", z.B. Verozellen), nicht beobachtet werden, was die Replikationsdefizienz von SeVV-eGFP-ΔP bestätigt (siehe Punkt 8).

### 5.5 Genexpression von SeVV-eGFP-ΔP in infizierten Zielzellen

Ein Ausbleiben der Vermehrung von SeVV-eGFP-ΔP auf Zelltypen, die das P Protein nicht *in trans* bereitstellen, konnte verifiziert werden. Gleichzeitig blieb die Expressionsleistung weit hinter den Erwartungen zurück.

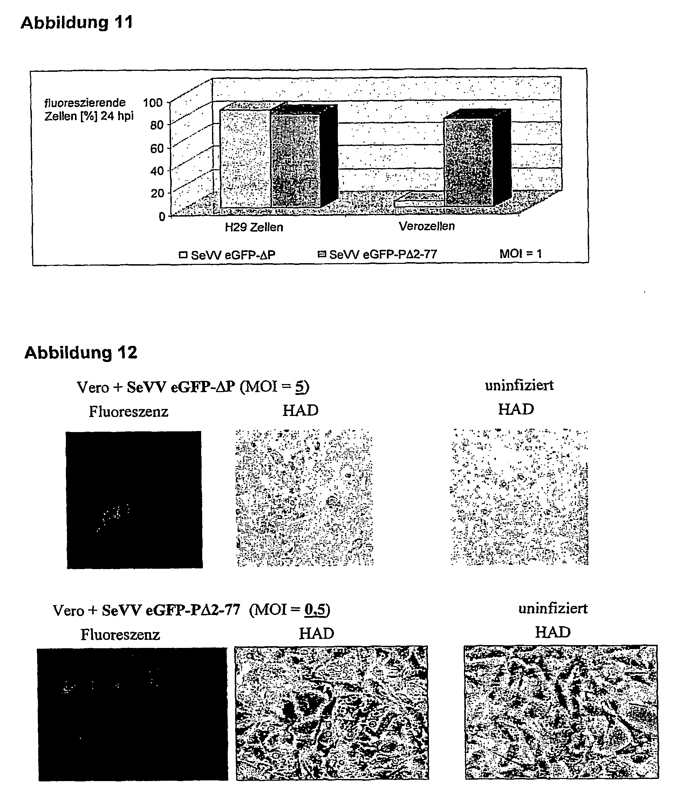

Ähnlich wie im Falle der Tollwutvirus ΔP Mutante (Shoji et al. (2004) Virology 318, 295-305) zeigten nur wenige infizierte Zellen eine schwache eGFP-Fluoreszenz (kleiner 5 %; siehe **Abbildung 11**), obwohl statistisch bei einer MOI = 1 tatsächlich ca. 70 % der Zellen mit je einem Viruspartikel infiziert sind. Auch bei einer höheren MOI = 5 sind nur vereinzelt grün leuchtende Verozellen durch SeVV-eGFP-ΔP zu beobachten (siehe **Abbildung 12**, oben links).

Dies bestätigt die Annahme, dass nach Infektion einer Zelle mit einem P Gen-defizienten Virus nur eine primäre Transkription über den mitgeführten Polymerasekomplex aus dem Viruspartikel möglich ist. Im Falle der SeV ΔP-Mutante sind außerdem anscheinend nur wenige Prozent der infizierenden Partikel dazu in der Lage bzw. ist eine Genexpression nur zu beobachten, wenn mehrere transkribierbare Nukleokapside gleichzeitig in einer Zelle vorliegen.

Für eine therapeutische Anwendung dieses replikationsdefizienten SeVV scheint die Expressionsleistung zu schwach bzw. es müssten unverhältnismäßig viele Partikel von SeW ΔP pro Patient appliziert werden. Daher ist es wünschenswert, eine replikationsdefiziente SeV Variante zu verwenden, die auch eine sekundäre Transkription erfüllt. Dies führt zur Entwicklung weiterer modifizierter Polymerasekomplexe, die das virale Genom nicht replizieren können, aber zu einer gesteigerten Expression des therapeutischen Gens bzw. Antigens in der Lage sind.

### 6. Herstellung eines modifizierten SeVV-eGFP-ΔP cDNA Konstrukts

Um die Transkriptionsleistung von P Gen-defizienten SeVV in der Zielzelle eventuell zu verbessern, wurde ein weiteres rekombinantes Konstrukt hergestellt, das an der Position des ursprünglichen P-Leserahmens für eine um 76 Aminosäuren N-terminal verkürzte Form des P-Proteins kodiert ("pSeVV-eGFP-PΔ2-77"; siehe Punkt 3.1 und **Abbildung 8B**).

Die Generierung von SeVV-eGFP-PΔ2-77 Partikeln und deren Vermehrung erfolgte analog zu Punkt 4.1 und 5.4.

### 6.1 Wachstumsverhalten von SeW-eGFP- PΔ2-77 in H29 Helferzellen

In SeVV-eGFP-PΔ2-77 infizierten H29 Helferzellen wird das N-terminal um 76 Aminosäuren verkürzte, viral-kodierte PΔ2-77-Protein zusammen mit dem zellulär kodierten P-Protein synthetisiert.

Um den Einfluss der Expression des verkürzten P-Proteins PΔ2-77 auf die virale Replikation zu untersuchen, wurde eine Infektion (MOI = 3) von H29 Zellen mit SeVV-eGFP-PΔ2-77, SeV-eGFP-ΔP oder dem Kontrollvirus SeV-eGFP analog zu dem unter Wachstumskinetik von SeVV-eGFP-ΔP beschriebenen Verfahren, durchgeführt. Die Überstände der einzelnen Ansätze wurden über einen Zeitraum von 120 h durch einen Zellinfektionsdosistest der Titer an Nachkommenviren bestimmt über die Zahl an eGFP-exprimierenden Zellen.

Aus einer SeV-eGFP infizierten H29-Zelle (Positivkontrolle) werden im Zeitraum von 120 h durchschnittlich 80 Viruspartikel freigesetzt, wobei eine Transkomplementation des P-Proteins durch H29 Zellen in diesem Fall nicht benötigt wurde. SeVV-eGFP-PΔ2-77 konnte im H29-Transkomplementationssystem mit etwa gleicher Effizienz wie SeVV-eGFP-ΔP vermehrt werden: Aus infizierten H29 Zellen werden nach 120 h etwa 20 x 10⁶ Viruspartikel von SeVV-eGFP-ΔP oder SeVV-eGFP-PΔ2-77 freigesetzt, dies entspricht einer Anzahl von etwa 40 freigesetzten Viruspartikeln der P-Mutanten pro H29 Zelle.

### 7. Vergleich der Genexpression von SeVV-eGFP-ΔP und SeVV- eGFP -PΔ2-77 und Quantifizierung der Proteinsynthese in infizierten Zielzellen

Um zu untersuchen, ob der Vektor SeVV-eGFP-PΔ2-77 eine verstärkte Transgenexpression - verglichen zu SeVV-eGFP-ΔP - in infizierten Zielzellen zeigt, wurde die viruskodierte Expression des Reportergens eGFP sowie des HN Proteins detailliert charakterisiert.

5 x 10⁵ Verozellen wurden mit SeVV-eGFP-PΔ2-77 infiziert (MOI = 1), wobei am Tag 2 p.i. ca. 70% fluoreszierende Verozellen beobachtet werden konnten (nicht gezeigt). Dies bedeutet, dass nahezu jeder RNP-Komplex dieser viralen Vektorvariante in der Lage ist, eine messbare Transkription in der Zielzelle zu induzieren.

### 7.1 Quantifizierung der eGFP-Expression durch FACS-Analyse

Die Transkriptionskassette, in die das Reportergen eGFP in SeVV-eGFP-PΔ2-77 eingesetzt wurde, soll in späteren Anwendungen im medizinischen Bereich das Antigen eines Pathogens, z.B. eines gewünschten Virus kodieren. Diese Antigenexpression muss ausreichend sein, um im Patienten eine protektive Immunantwort hervorzurufen.

Um sicherzustellen, dass jedes SeVV-eGFP-PΔ2-77 Nukleokapsid, das eine Zielzelle infiziert, in der Lage ist, eine detektierbare Transgenexpression durchzuführen, sollte die gleiche Anzahl an H29 und Verozellen mit derselben Menge an Viruspartikeln infiziert werden und mittels FACS (*fluorescent activated cell sorting*)-Analyse unter Verwendung eines FACS-Calibur Durchflusszytometers die Anzahl der eGFP-exprimierenden H29- bzw. Verozellen verglichen werden. Die Daten wurden per Computerhistogramm ausgewertet, indem die Fluoreszenzsignale infizierter Zellen gegen die Gesamtzellzahl aufgetragen wurden.

2,5 x 10⁵ Verozellen oder H29 Zellen wurden mit SeVV-eGFP-PΔ2-77 oder dem P Gen-defizienten Virus SeVV-eGFP-ΔP mit einer MOI = 1 infiziert. Die FACS-Analyse der Proben erfolgte 24 h nach Infektion. Die infizierten Zellen wurden in PBS aufgenommen und durchflusszytometrisch die Anzahl fluoreszierender Zellen ermittelt. Das Ergebnis ist in **Abbildung 11** als Anteil [%] fluoreszierender Vero- und H29 Zellen angegeben.

24 h nach Infektion von H29 Helferzellen mit SeVV-eGFP-ΔP konnten 86 % eGFP-exprimierende H29 Zellen durch FACS-Analyse detektiert werden. Hier unterstützt die zelluläre Synthese des P-Proteins die Bildung neuer P:L-Komplexe und damit Produktion neuer mRNA, was zur Synthese von eGFP-Proteinen in der infizierten Zelle führt. Wurden dagegen Verozellen mit SeVV-eGFP-ΔP infiziert, konnte weder 24 h p.i. noch zu einem späteren Zeitpunkt in weiteren Versuchsansätzen eine Expression des eGFP-Proteins nachgewiesen werden. Die durch SeVV-eGFP-ΔP Nukleokapside transferierten P:L-Komplexe sind bei einer Infektion mit MOI = 1 nicht in der Lage, eine detektierbare Expression in infizierten Verozellen herbeizuführen.

24 h nach SeVV-eGFP-PΔ2-77 Infektion konnten dagegen durch FACS-Analyse knapp 80 % eGFP-exprimierende H29 Zellen und ebenso 75 % eGFP-exprimierende Verozellen identifiziert werden. Bei der Infektion von H29 Zellen kann die Transkription der eGFP mRNA durch die zelluläre Synthese des P-Proteins und damit durch neue P:L-Komplexe unterstützt werden. Bei der Infektion von Verozellen mit SeVV-eGFP-PΔ2-77 wird die Transkription durch die Neusynthese des N-terminal verkürzten P-Proteins PΔ2-77 verstärkt.

Aus diesen Ergebnissen kann eine wichtige Aussage über die Anzahl transkriptionsfähiger SeVV-eGFP-PΔ2-77 getroffen werden: H29 Zellen und Verozellen wurden mit einer MOI von 1 infiziert. Theoretisch auftretende Mehrfachinfektionen einer Zelle sind in beiden Ansätzen statistisch gleich wenig wahrscheinlich. 24 h p.i. konnten knapp 80 % eGFP-exprimierende H29 Zellen und etwa 75 % eGFP-exprimierender Verozellen identifiziert werden. Dadurch lässt sich ableiten, dass jeder RNP-Komplex mit der verkürzten P-Variante PΔ2-77, welcher zur Transgenexpression in einer P-Helferzelle fähig ist, ebenso eine Transgenexpression in der infizierten Zielzelle erbringt. Im Gegensatz dazu erfüllt die Variante SeVV-eGFP-ΔP mit einer kompletten Deletion des P ORF diese Eigenschaft nicht.

### 7.2 Funktionstest des in infizierten Zielzellen exprimierten HN-Proteins

Mit Hilfe eines Hämadsorptions (HAD)-Tests wurde die Effizienz der Bindung humaner Erythrozyten und damit die Effizienz der Exposition viraler HN-Proteine auf einzelnen infizierten Zellen detektiert (**Abbildung 12**).

5 x 10⁵ Verozellen wurden mit SeVV-eGFP-PΔ2-77 bei einer niedrigen MOI = 0,5 infiziert. Im Gegensatz dazu mussten Verozellen im Falle von SeVV-eGFP-ΔP mit einer 10fach höheren MOI = 5 infiziert werden, um überhaupt einzelne fluoreszierende Zellen beobachten zu können. Nach 1-stündiger Adsorption erfolgte ein Mediumwechsel mit DMEM + 10% FKS. Anschließend wurden die Zellen mehrere Tage bei 33 °C inkubiert. Die Transgenexpression beider Vektorvarianten wurde zunächst wieder über die eGFP-Fluoreszenz verfolgt. Am Tag 5 und 9 p.i. wurden HAD-Testreihen durchgeführt, um die Exposition der viralen HN-Proteine anhand der Bindung humaner Erythrozyten zu analysieren.

Obwohl im Falle von SeVV-eGFP-ΔP bei einer MOI = 5 statistisch 99,3 % der Verozellen infiziert sein sollten, konnten nur 0,01 % eGFP-positive Zellen unter dem Mikroskop beobachtet werden (**Abbildung 12** oben links). Dagegen ist mit SeVV-eGFP-PΔ2-77 eine grüne Fluoreszenz bei einer MOI = 0,5 erwartungsgemäß in 40 % der Zellen zu sehen (**Abbildung 12** unten links).

Zwei Tage nach Infektion mit SeVV-eGFP-ΔP waren nur die Hälfte der eGFP-positiven Zellen (25 von 5x 10⁵ infizierten) in der Lage, Erythrozyten an ihrer Oberfläche zu binden (**Abbildung 12** oben mitte). Nach weiterer Inkubation und nochmaligem HAD-Test fand keine Erythrozyten-Adsorption mehr an infizierte Zellen statt. Mit der zweiten replikationsdefizienten SeVV Variante SeVV-eGFP-PΔ2-77 konnten wiederum wesentlich bessere Ergebnisse erzielt werden: 5 Tage nach Infektion waren ca. 40 % der infizierten Zellen (MOI = 0,5) in der Lage, Erythrozyten an ihrer Oberfläche zu binden (**Abbildung 12** unten mitte). Dabei konnte bei den einzelnen Zellen eine unterschiedliche Bindungsaktivität von 10 bis 70 komplexierten roten Blutkörperchen beobachtet werden. Damit war gezeigt, dass die mit SeVV-eGFP-PΔ2-77 infizierten Zellen 5 d p.i. in der Lage sind, HN-Proteine auf der Zelloberfläche zu exponieren, der funktionelle HAD-Test kann als positiv bewertet werden. Gleichzeitig konnte anhand der unterschiedlichen Menge gebundener Erythrozyten eine effiziente Expression des HN-Proteins in den infizierten Zellen ermittelt werden.

Die infizierten Zellen wurden bei 33°C weiter inkubiert, wobei die Neuraminidase-Aktivität des HN-Proteins das Ablösen der an infizierte Zellen gebundenen Erythrozyten bewirkt. Die Zellen wurden gewaschen, um die abgelösten Erythrozyten zu entfernen, und für weitere 4 d bei 33°C inkubiert. Am Tag 9 p.i. wurde wiederum ein HAD-Test durchgeführt. Es konnten nun etwa 30 % HAD-positive Verozellen detektiert werden. Dabei ging die Anzahl gebundener roter Blutkörperchen auf 5 bis 20 Erythrozyten pro Zelle zurück.

Es wurde also 9 Tage i.p. immer noch genügend funktionelles HN durch die replikationsdefiziente Variante SeVV-eGFP-PΔ2-77 synthetisiert. Im Gegensatz dazu erfüllt die Variante SeVV-eGFP-ΔP mit einer kompletten Deletion des P ORF diese Eigenschaften nicht.

### 7.3 Quantifizierung der eGFP-Expression durch Western Blot-Analyse

Es wurde eine semi-quantitative Abschätzung der eGFP-Expression des replikationsdefizienten SeV-Vektors im Vergleich zu dem replikationskompetenten SeV-eGFP durch eine Western Blot-Analyse mittels seriellen Verdünnungsreihen von zellulärem Gesamtprotein durchgeführt.

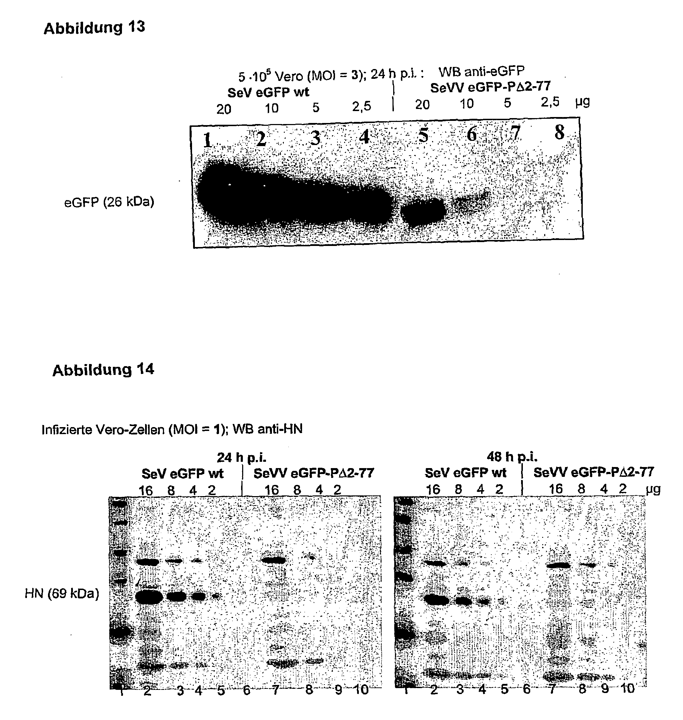

5 x 10⁵ Verozellen wurden mit SeV-eGFP oder SeVV-eGFP-PΔ2-77 infiziert (MOI = 3). 24 h p.i. erfolgte der Aufschluss der Zellen; die Zellextrakte wurden in seriellen Verdünnungsreihen (1:2) von 20 µg bis 2,5 µg-Gesamtmenge in einem SDS-PAGE aufgetrennt. Die Proteine wurden auf eine PVDF-Membran transferiert und zunächst das viral kodierte eGFP-Protein (26 kDa) durch Western Blot-Analyse nachgewiesen (**Abbildung 13**).

Das Fluoreszenzprotein eGFP konnte sowohl in SeV-eGFP als auch in SeVV-eGFP-PΔ2-77 infizierten Verozellen mit Hilfe der Western Blot-Analyse nachgewiesen werden. Ein Vergleich der Intensitäten der eGFP-Signale lässt die Aussage zu, dass die Expression - vermittelt über den replikationsdefizienten SeVV-eGFP-PΔ2-77 - um etwa Faktor 16 reduziert ist im Vergleich zum replikationskompetenten SeV-eGFP .

Eine Reduktion um Faktor 16 ist dabei gering und lässt die Aussage zu, dass SeVV-eGFP-PΔ2-77 trotz des modifizierten P-Proteins eine sehr effiziente sekundäre Transkription erbringen kann.

### 7.4 Abschätzung der HN-Expression durch Western Blot-Analyse

Das SeV HN-Protein ist, im Gegensatz zum eGFP-Protein, ein membranständiges Oberflächenprotein und stellt eine wichtige Antigen-Determinante dar. Durch relative Quantifizierung der Expressionsstärke des HN-Proteins in SeVV-eGFP-PΔ2-77 infizierten Zellen wurde eine Aussage über die Intensität der Expression des SeV HN-Antigens ermöglicht.

Es wurde eine semi-quantitative Abschätzung der HN-Expression des replikationsdefizienten SeV-Vektors im Vergleich zu dem replikationskompetenten SeV-eGFP durch eine Western Blot-Analyse mittels seriellen Verdünnungsreihen von zellulärem Gesamtprotein durchgeführt. Je 5 x 10⁵ Verozellen wurden in 2 parallelen Ansätzen mit SeV-eGFP oder SeVV-eGFP-PΔ2-77 infiziert (MOI = 1) und 24 h bzw. 48 h inkubiert. Danach erfolgte der Aufschluss der Zellen; die Zellextrakte wurden in seriellen Verdünnungsreihen (1:2) von 16 µg bis 2 µg Gesamtmenge in einem SDS-PAGE aufgetrennt. Die Proteine wurden auf eine PVDF-Membran transferiert und das viral kodierte HN-Protein (60 kDa) mit Hilfe eines monoklonalen HN-Antikörpers nachgewiesen (**Abbildung 14**).

Das HN-Protein konnte bei SeV-eGFP infizierten Verozellen nach beiden Inkubationszeiten in allen Spuren (16 bis 2 µg Gesamtprotein; Spuren 2-5 links und rechts) effizient detektiert werden. Im Falle von SeVV-eGFP-PΔ2-77 ist die Bande des HN-Proteins noch in den Spuren mit 16 und 8 µg Gesamtprotein (Spur 7, 8) sichtbar, jedoch mit schwächerer Intensität. Die relative Quantifizierung der HN-Expression in SeVV-eGFP-PΔ2-77 infizierten Verozellen gegenüber SeV-eGFP wurde durch den Vergleich der Spuren mit 16 und 8 µg vs. 2 µg Gesamtprotein vorgenommen (Spuren 7 und 8 vs. 5, links und rechts) und erlaubt eine geschätzte Reduktion der HN-Expression um Faktor 8 - 16 der P-Deletionsvariante, unabhängig von der Inkubationszeit. Dies lässt auf eine relativ hohe Transkriptionsrate in SeVV-eGFP-PΔ2-77 infizierten Zielzellen schließen und damit auf eine generell hohe Transgenexpression des viralen replikationsdefizienten Vektors.

Unter Einbeziehung beider Messungen (eGFP- und HN-Protein) kann von einer durchschnittlichen Reduktion der Expression um Faktor 10 ausgegangen werden.

### 8. Replikationsdefizienz von SeVV-eGFP-PΔ2-77 in der Zielzelle

Werden Verozellen mit dem replikationskompetenten SeV-eGFP infiziert, entsteht in den zwei darauf folgenden Tagen um die initial infizierte, stark fluoreszierende Zelle ein Spot aus bis zu tausend weiteren fluoreszierenden Zellen. Um die Replikationsdefizienz von SeVV-eGFP-PΔ2-77 in Verozellen zu beweisen, sollte nun das Ausbleiben dieser Zunahme grün fluoreszierender Zellen um eine initial infizierte Zielzelle unter Berücksichtigung der natürlichen Teilungsrate von Verozellen bestätigt werden. Verozellen teilen sich im Durchschnitt alle 24 h. Werden Verozellen mit SeVV-eGFP-PΔ2-77 infiziert, ist eine detektierbare eGFP-Expression nach etwa 24 h zu sehen. Es wurde beobachtet, dass nach weiteren 24 h Inkubationsphase aus dieser initial infizierten, fluoreszierenden Verozelle aufgrund natürlicher Teilung in einigen Fällen zwei (schwächer) fluoreszierende Tochterzellen hervorgehen. Diese Beobachtung hat nichts mit einer Virusvermehrung zu tun, bei der zwischen 10¹ bis 10⁴ Viruspartikel aus einer infizierten Zielzelle entlassen werden, die dann nebenliegende Zellen infizieren können. Diese natürliche Teilungsrate infizierter Zellen hat andererseits einen Einfluss auf die eGFP-Expressionsstärke, die mit zunehmender Anzahl von Zellteilungen reduziert wird. Diese Beobachtung zeigt, dass der virale Vektor SeVV-eGFP-PΔ2-77 Genomreplikationsdefizient ist, also keine neuen Genome synthetisiert werden. Führen mehrere aufeinander folgende Zellteilungen einer infizierten Zelle zu einer kontinuierlichen Abnahme der Intensität der Fluoreszenz, bis diese schließlich zum Erliegen kommt, so ist eine Virusvermehrung auszuschließen.

Um die Replikationsdefizienz von SeVV-eGFP-PΔ2-77 in Zielzellen endgültig zu bestätigen, wurde eine letzte Untersuchung durchgeführt: Es wurde eine T75-Flasche mit - 20 x 10⁶ Verozellen ausgelegt. Die Zellen waren schon zu Beginn der Inkubationsphase in hoher Dichte ausgesät und demzufolge nicht mehr stark teilungsaktiv. Diese Verozellen wurden mit SeVV-eGFP-PΔ2-77 mit einer MOI von 0,001 infiziert. Ein Mediumwechsel nach DMEM mit 5 % FKS (für verringerte Teilungsaktivität) erfolgte nach einstündiger Inkubationszeit und die Verozellen wurden bei 33 °C inkubiert (P1). Zwei Tage p.i. konnten, entsprechend der gewählten MOI, initial mehrere tausend einzeln liegende infizierte, fluoreszierende Verozellen beobachtet werden. Wegen der hohen Zelldichte kam es in den darauf folgenden 4 Inkubationstagen kaum zu Zellteilungen, d.h. die Anzahl initial infizierter Zellen, detektiert durch die Fluoreszenz, blieb konstant. Würden in dieser Zeit Viruspartikel gebildet worden sein, hätten diese nebenliegende Zellen infizieren können, was sich an einer Zunahme der Fluoreszenz widergespiegelt hätte. Auch nach 8 Tagen konnte eine Ausbreitung des viralen Vektors wegen fehlender Neuinfektionen umliegender Zellen ausgeschlossen werden. Um die Zellen mit frischem Medium zu versorgen, wurde der Überstand abgenommen und die Verozellen mit neuem Medium überschichtet. 12 Tage nach Inkubationsstart lösten sich die Verozellen vom Kulturboden ab. Während der gesamten Versuchsdauer konnte keine Replikation des viralen Vektors in Form einer Zunahme fluoreszierender Zellen beobachtet werden. Eine Ausbreitung von SeVV-eGFP-PΔ2-77 von den primär infizierten Zellen auf umliegende Verozellen durch Produktion neuer Virusgenome sowie -partikel konnte somit ausgeschlossen werden. Daher ist SeVV-eGFP-PΔ2-77 als ein replikationsdefizienter viraler Vektor zu bezeichnen.

### Fazit:

Die oben aufgeführten Ergebnisse zeigen, dass durch gezielte Manipulationen an Genen für Komponenten des Polymerase-Komplexes replikationsdefiziente Negativstrang RNA-Viren hergestellt werden können, die nur noch in der Lage sind, die viruskodierten Gene zu transkribieren, aber das Virusgenom nicht mehr zu replizieren.

Im Falle des Sendaivirus wurden speziell zwei Varianten näher untersucht, in denen das Gen für den Polymerase-Cofaktor Phosphoprotein entweder komplett deletiert wurde ("SeVV-eGFP-ΔP") oder die Codons für die Aminosäuren 2 bis 77 entfernt wurden ("SeVV-eGFP-PΔ2-77"). Beide SeV Vektoren sind replikationsdefizient in Zellen, die das P Protein nicht *in trans* zur Verfügung stellen (sog. Zielzellen), unterscheiden sich jedoch erheblich in ihrer Genexpressionsleistung.

Obwohl im Falle von SeVV-eGFP-ΔP bei einer MOI = 5 statistisch nur 0,7 % der Verozellen uninfiziert bleiben - 99,3 % sollten mindestens einen RNP-Komplex enthalten - konnten nur 0,01 % eGFP-positive Zellen unter dem Mikroskop beobachtet werden. Daraus kann rechnerisch gefolgert werden, dass es nur dann zu einer sichtbaren Transgenexpression kommt, wenn 15 oder mehr RNPs von SeVV-eGFP-ΔP gleichzeitig in einer infizierten Zielzelle vorhanden sind.

Dieser P Gen-defiziente SeVV zeigt eine ähnlich schwache Expression wie die analoge Tollwut ΔP-Variante (Shoji et al., supra). Beide Vektoren sind in der infizierten Zielzelle nur zur primären Transkription über den mitgeführten Polymerasekomplex aus dem Viruspartikel fähig. Für eine therapeutische Anwendung des Vektors ist jedoch eine stärkere Expression des kodierten Transgens bzw. Antigens erwünscht. Diese Bedingung kann mit Hilfe der replikationsdefizienten Variante SeVV-eGFP-PΔ2-77 erfüllt werden, die in Zielzellen eine nur um durchschnittlich Faktor 10 reduzierte Expressionsleistung erbringt im Vergleich zum replikationskompetenten SeV. Durch die Anwesenheit des Gens für ein N-terminal verkürztes P Protein im Vektorgenom ist nicht nur eine primäre, sondern auch eine sekundäre Transkription möglich. Diese wird bewerkstelligt durch neu entstandene, modifizierte Polymerase-Komplexe, die das Vektor-kodierte PΔ2-77 Protein enthalten; dieses unterstützt jedoch nicht den Replikationsmodus der Polymerase.

Durch die Quantifizierung der Proteinsynthese in infizierten Zielzellen ist bewiesen, dass der replikationsdefiziente virale Vektor SeVV-eGFP-PΔ2-77 in der Lage ist, eine effiziente Transkription und Expression viral kodierter Gene durchzuführen. Dabei wird nicht nur das 3'-proximale Transgen (eGFP) effektiv synthetisiert; auch das an Genomposition 6 liegende HN-Gen wird über mindestens 9 Tage nach Infektion transkribiert und das Protein funktionell auf infizierten Zielzellen exponiert.

### 9. Bestimmung der durch eine replikationsdefiziente RNA-Vakzine in einem Mausmodell induzierten Immunantwort

Es konnte gezeigt werden, dass vorzugsweise durch eine Deletion im P-Gen ("PΔ2-77") ein veränderter viraler Polymerase-Komplex entsteht, der keine Synthese neuer Genome mehr zulässt. Gleichzeitig ist nach Infektion mit solchen replikationsdefizienten Viren die in der Zielzelle vermittelte virale Genexpression nur ca. 10x niedriger im Vergleich zu Infektionen mit replikationskompetentem Virus.

Um eine hinreichend immunogene Eigenschaft des replikationsdefizienten Negativstrang RNA-Virus als Vakzinierungsvektor nachzuweisen, wurden Antigene bzw. antigene Determinanten zweier heterologer Viren (humanes Parainfluenzavirus Typ 3, hPIV3, und Respiratorisches Synzytialvirus, RSV) in das Virusgenom eingesetzt: Dazu wurde ein replikationsdefizientes SeV PΔ2-77 konstruiert, in dem die Gene der ursprünglichen Oberflächenproteine F und HN ausgetauscht sind gegen Gene, die für chimäre F und HN Proteine SeV/hPIV3 kodieren. Das chimäre F-Protein enthält 558 Aminosäuren und besteht aus der extrazellulären Domäne von hPIV3 (493 Aminosäuren), der Transmembrandomäne von SeV (23 Aminosäuren) und der cytoplasmatischen Domäne von SeV (42 Aminosäuren). Das chimäre HN-Protein hat 579 Aminosäuren und besteht aus der cytoplasmatischen Domäne von SeV (35 Aminosäuren), der Transmembrandomäne von SeV (25 Aminosäuren) sowie der extrazellulären Domäne von hPIV3 (519 Aminosäuren). Die Aminosäuresequenzen des chimären F-Proteins und des chimären HN-Proteins sind im Sequenzprotokoll als SEQ ID No. 27 und 28 gezeigt.

Durch Insertion chimärer Gene in das Virusgenom wird eine neue Antigenität erzeugt und zugleich der effiziente Zusammenbau von Vakzinpartikeln bei deren Produktion sichergestellt.

In einer zusätzlichen Expressionskassette eingeschoben zwischen zwei viralen Genen erfolgte die Kodierung des Oberflächenproteins F von RSV, wodurch das Konstrukt zu einer bivalenten Vakzine erweitert wurde.

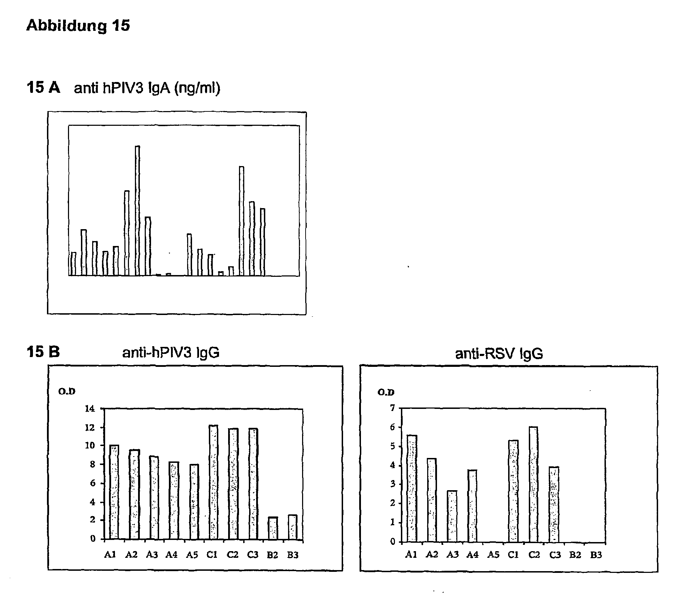

Diese neue Vakzine wurde im Tiermodell getestet. Es wurden Gruppen von Balb/C Mäusen dreimal im Abstand von drei Wochen mit zwei verschiedenen Viruspräparationen (Gruppe A bzw. C, je 10⁴ infektiöse Einheiten) intranasal immunisiert, eine Kontrollgruppe (B) erhielt PBS anstelle der Vakzine. Nach der dritten Immunisierung wurden zur Analyse der mukosalen Immunantwort nasale Waschflüssigkeit (NW) gewonnen sowie bronchoalveoläre Spülungen (BAL) durchgeführt und zur Analyse der humoralen Immunantwort das Serum isoliert. Über ELISA wurde die Menge an induzierten Immunglobulinen IgA und IgG spezifisch gegen hPIV3 sowie RSV bestimmt. Der replikationsdefiziente Vakzinprototyp bewirkte eine deutliche Induktion von IgA-Antikörpern spezifisch gegen hPIV3 (Abb. 15A), die Induktion von anti-RSV IgA-Antikörpern fiel geringer aus (nicht gezeigt). Die Induktion einer humoralen Immunantwort gegen die OberflächenAntigene beider Viren ergab vergleichbare Titer, die Menge an spezifischen IgG unterscheidet sich um Faktor 2 (Abb. 15B). Eine weiterführende Analyse des anti-hPIV3-IgG zeigte, dass die induzierten Antikörper neutralisierende Eigenschaften (Titer 1/64) aufweisen. Im Gegensatz dazu konnte - wie erwartet - bei der Kontrollgruppe keinerlei spezifische IgA- bzw. IgG-Induktion festgestellt werden.

Die erfindungsgemäße Vakzine konnte eine spezifische mukosale und humorale Immunantwort gegen heterologe virale Antigene induzieren. Zusätzliche Experimente zeigten, dass Lymphozyten immunisierter Mäuse Interferon-γ produzierten, während IL-5 nicht nachgewiesen werden konnte. Dieser Befund deutet darauf hin, dass die bivalente, replikationsdefiziente RNA-Vakzine in der Lage ist, eine T-Zellimmunantwort auszulösen, welche eine Voraussetzung für eine lang anhaltende Immunität ist.

### Fazit

Nach Infektion von Versuchstieren mit einem modifizierten Vektor, in den kodierende Sequenzen für Antigene zweier heterologer Viren eingesetzt waren, wurde die Induktion neutralisierender Antikörper nachgewiesen. Dies zeigt das Potential von replikationsdefizienten Negativstrang RNA-Viren für die Entwicklung von neuartigen Impfstoffen.

### 10. Aufstellung der verwendeten DNA-Oligonukleotide

Die in den obigen Beispielen verwendeten DNA-Oligonukleotide sind in der folgenden Tabelle 3 angegeben.

**Tabelle 3:**

| **SEQ ID No.** | **Bezeichnung** | **Länge [nt]** | **Sequenz 5'→3'** | **Tₘ [°C]** |
|---|---|---|---|---|
| 1 | XI=M13 | 19 | GGAAACAGCTATGACCATG | 54 |
| 2 | XI (+) | 59 | | 58 |
| 3 | XII | 57 | | 64 |
| 4 | XII(+) (+) = N-1029(+) | 18 | GGTAGGTGTCTATGAGGC | 56 |
| 5 | XX | 44 | | 61 |
| 6 | XX (+) | 57 | | 61 |
| 7 | *NotI* eGFP | 44 | | 60 |
| 8 | eGFP *Notl* (+) | 26 | GATGCATGCTCGAGCGGCCGCTTTAC | 58 |
| 9 | *SgrAI* eGFP | 36 | | 61 |
| 10 | eGFP *SgrAl* (+) | 37 | | 63 |
| 11 | *Fsel* DsRed | 36 | | 59 |
| 12 | DsRed *Fsel* (+) | 42 | | 63 |
| 17 | ΔP I = N-578 | 17 | CCCTGACACACTCCTTC | 54 |
| 18 | ΔP I (+) | 31 | GCGCCGCTCGAGGCGGTAAGTGTAGCCGAAG | 64 |
| 19 | ΔP II | 34 | | 64 |
| 20 | ΔP II(+) | 34 | GGCGACGCGTCAGTCTCACAGCCTAATTCG | 64 |
| 21 | *XhoI* PΔ2-77 | 47 | | 84 |
| 22 | PΔ2-77 (+) *XhoI* | 46 | | 80 |

## Patentansprüche

1. Rekombinantes Negativstrang RNA-Virus, enthaltend ein virales Genom mit einer Mutation in dem Gen P, die zum Verlust der Replikationsfähigkeit ohne Verlust der sekundären Transkriptionsfähigkeit führt.

2. Virus nach Anspruch 1,
**dadurch gekennzeichnet,**
**dass** es ein Paramyxovirus ist.

3. Virus nach Anspruch 1 oder 2,
**dadurch gekennzeichnet,**
**dass** es ein Sendaivirus ist.

4. Virus nach Anspruch 1,
**dadurch gekennzeichnet,**
**dass** die Mutation die N-terminale Teilsequenz des vom Gen P kodierten Proteins betrifft.

5. Virus nach Anspruch 4,
**dadurch gekennzeichnet,**
**dass** die Mutation umfasst: eine Deletion von
(a) den Aminosäuren 2-77 des vom Gen P kodierten Proteins oder
(b) einer zum Verlust der Replikationsfähigkeit ausreichenden Teilsequenz von (a).

6. Virus nach einem der Ansprüche 1 bis 5,
**dadurch gekennzeichnet,**
**dass** das virale Genom mindestens eine für ein heterologes Genprodukt kodierende Sequenz enthält.

7. Virus nach Anspruch 6,
**dadurch gekennzeichnet,**
**dass** das heterologe Genprodukt ein Protein, ein Ribozym, ein Antisense-Molekül oder ein siRNA-Molekül ist.

8. Virus nach Anspruch 6 oder 7,
**dadurch gekennzeichnet,**
**dass** das heterologe Genprodukt ein Reporterprotein, ein Antigen oder ein therapeutisches Protein ist.

9. Virus nach einem der Ansprüche 6 bis 8,
**dadurch gekennzeichnet,**
**dass** das heterologe Genprodukt ein Antigen eines heterologen Pathogens, ausgewählt aus Viren, Bakterien und Protozoen, ist.

10. Virus nach einem der Ansprüche 6 bis 9,
**dadurch gekennzeichnet,**
**dass** das heterologe Genprodukt ein virales Antigen ist.

11. Virus nach Anspruch 10,
**dadurch gekennzeichnet,**
**dass** das virale Genom für mehrere heterologe Antigene aus gleichen oder verschiedenen Viren kodiert.

12. Virus nach einem der Ansprüche 6 bis 11,
**dadurch gekennzeichnet,**
**dass** die für mindestens ein heterologes Genprodukt kodierende Sequenz in das viral Genom inseriert ist oder/und für ein homologes Genprodukt kodierende Sequenzen substituiert.

13. Virus nach einem der Ansprüche 1 bis 12,
**dadurch gekennzeichnet,**
**dass** das Virus im Vergleich zum Wildtyp eine um höchstens den Faktor 20 verringerte Transkriptionsfähigkeit aufweist.

14. Nukleokapsid eines Negativstrang RNA-Virus nach einem der Ansprüche 1 bis 13.

15. Genom eines Negativstrang RNA-Virus nach einem der Ansprüche 1 bis 13.

16. DNA-Molekül, das für das Genom oder/und Antigenom eines rekombinanten Negativstrang RNA-Virus nach einem der Ansprüche 1 bis 13 kodiert.

17. DNA-Molekül nach Anspruch 16,
**dadurch gekennzeichnet,**
**dass** es in operativer Verknüpfung mit einem Transkriptionssignal steht.

18. DNA-Molekül nach Anspruch 17,
**dadurch gekennzeichnet,**
**dass** das Transkriptionssignal ein Bakteriophagenpromotor, z.B. ein T7 oder SP6-Promotor, ist.

19. Zelle, die ein Virus nach einem der Ansprüche 1 bis 13, ein Nukleokapsid nach Anspruch 14, ein Genom nach Anspruch 15 oder ein DNA-Molekül nach einem der Ansprüche 16 bis 18 enthält.

20. Zelle nach Anspruch 19,
**dadurch gekennzeichnet,**
**dass** sie eine Vektor-Vermehrungszelle ist.

21. Zelle nach Anspruch 19,
**dadurch gekennzeichnet,**
**dass** sie eine Virus-Herstellungszelle ist.

22. Zelle nach Anspruch 21,
**dadurch gekennzeichnet,**
**dass** sie weiterhin ein für eine heterologe DNA-abhängige RNA Polymerase kodierendes DNA-Molekül enthält, welches die Transkription des für das rekombinante Negativstrang RNA-Virus kodierenden DNA-Moleküls bewirkt.

23. Zelle nach Anspruch 19,
**dadurch gekennzeichnet,**
**dass** sie eine Virus-Vermehrungszelle ist.

24. Zelle nach einem der Ansprüche 19 bis 23,
**dadurch gekennzeichnet,**
**dass** sie weiterhin für das virale P-Protein kodierende DNA-Moleküle enthält.

25. Verfahren zur Herstellung eines Negativstrang RNA-Virus nach einem der Ansprüche 1 bis 14, umfassend die Schritte:
(a) Bereitstellen einer Zelle, die mit einem DNA-Molekül transfiziert ist, das für das Genom eines Negativstrang RNA-Virus kodiert, enthaltend eine Mutation in dem Gen P, die zum Verlust der viralen Genom-Replikationsfähigkeit ohne Verlust der sekundären Transkriptionsfähigkeit führt, und gegebenenfalls mindestens eine für ein heterologes Genprodukt kodierende Sequenz, und
(b) Kultivieren der Zelle unter Bedingungen, dass eine Transkription der DNA gemäß (a) erfolgt und das rekombinante Negativstrang RNA-Virus gebildet wird.

26. Verfahren nach Anspruch 25,
weiterhin umfassend das Gewinnen des Nukleokapsids oder des viralen Genoms aus dem Negativstrang RNA-Virus.

27. Verfahren zur Vermehrung eines Negativstrang RNA-Virus nach einem der Ansprüche 1 bis 13, umfassend die Schritte:
(a) Bereitstellen einer Zelle, die mit einem Negativstrang RNA-Virus infiziert ist, enthaltend eine Mutation in dem Gen P, die zum Verlust der viralen Genom-Replikationsfähigkeit ohne Verlust der sekundären Transkriptionsfähigkeit führt, und gegebenenfalls mindestens eine für ein heterologes Genprodukt kodierende Sequenz, und
(b) Kultivieren der Zelle unter Bedingungen, dass eine Vermehrung des Virus erfolgt.

28. Pharmazeutische Zusammensetzung,
**dadurch gekennzeichnet,**
**dass** sie ein rekombinantes Negativstrang RNA-Virus nach einem der Ansprüche 1 bis 13, ein Nukleokapsid nach Anspruch 14 oder ein virales Genom nach Anspruch 15 als Wirkstoff sowie gegebenenfalls pharmazeutisch übliche Träger- oder/und Hilfsstoffe enthält.

29. Pharmazeutische Zusammensetzung nach Anspruch 28 zur Anwendung als Impfstoff.

30. Pharmazeutische Zusammensetzung nach Anspruch 29 als mono-oder polyvalenter Impfstoff.

31. Pharmazeutische Zusammensetzung nach Anspruch 29 oder 30 als Impfstoff gegen virale Infektionen, beispielsweise als Impfstoff gegen Infektionen mit pathogenenen Negativstrang RNA-Viren.

32. Pharmazeutische Zusammensetzung nach Anspruch 28 zur Anwendung für Anti-Tumor-Therapie.

33. Pharmazeutische Zusammensetzung nach einem der Ansprüche 28 bis 32 zur Anwendung bei Risikopatienten.

34. Pharmazeutische Zusammensetzung nach einem der Ansprüche 28 bis 33, umfassend das Nukleokapsid in der nativen viralen Hülle.

35. Verwendung einer Zelle, die konstitutiv oder induzierbar das Protein P eines Negativstrang RNA-Virus stabil exprimiert, zur Herstellung oder Vermehrung von rekombinanten Negativstrang RNA-Viren nach einem der Ansprüche 1 bis 13, von Nukleokapsiden nach Anspruch 14 oder von viralen Genomen nach Anspruch 15.

36. Verwendung nach Anspruch 35,
**dadurch gekennzeichnet,**
**dass** die Zelle eine Säugerzelle ist.

37. Verwendung nach Anspruch 35 oder 36,
**dadurch gekennzeichnet,**
**dass** die Zelle ausgewählt ist aus der Zelle H29 (DSM ACC2702) oder einer davon abgeleiteten Zelle.

## Claims

1. Recombinant negative-strand RNA-virus, comprising a viral genome with a mutation in the P-gene, which results in a loss of replicability without the loss of secondary transcription-capability.

2. Virus according to claim 1, **characterized in that** it is a Paramyxovirus.

3. Virus according to claim 1 or 2, **characterized in that** it is a Sendai virus.

4. Virus according to claim 1, **characterized in that** the mutation effects the N-terminal partial sequence of the protein encoded by the P-gene.

5. Virus according to claim 4, **characterized in that** the mutation comprises: a deletion of
(a) the aminoacids 2-77 of the protein encoded by the P-gene or
(b) a partial sequence of (a) which is sufficient to result in a loss of replicability.

6. Virus according to any one of the claims 1 to 5, **characterized in that** the viral genome contains at least one sequence encoding for a heterologous gene product.

7. Virus according to claim 6, **characterized in that** the heterologous gene product is a protein, a ribozyme, an antisense-molecule or a siRNA-molecule.

8. Virus according to claim 6 or 7, **characterized in that** the heterologous gene product is a reporter protein, an antigen or a therapeutic protein.

9. Virus according to any one of the claims 6 to 8, **characterized in that** the heterologous gene product is an antigen of an heterologous pathogen, selected from viruses, bacteria and protozoa.

10. Virus according to any one of the claims 6 to 9, **characterized in that** the heterologous gene product is a viral antigen.

11. Virus according to claim 10, **characterized in that** the viral genome codes for several heterologous antigens derived from the same or different viruses.

12. Virus according to any one of the claims 6 to 11, **characterized in that** at least one of the sequences encoding for a heterologous gene product is inserted into the viral genome and/or substitutes sequences which code for a homologous gene product.

13. Virus according to any one of the claims 1 to 12, **characterized in that** the virus shows a transcription-capability reduced at most by an factor of 20 in comparison to the wild-type.

14. Nucleocapsid of a negative-strand RNA-virus according to any one of the claims 1 to 13.

15. Genome of a negative-strand RNA-virus according to any one of the claims 1 to 13.

16. DNA-molecule which codes for the genome and/or the anti-genome of a recombinant negative-strand RNA-virus according to any one of the claims 1 to 13.

17. DNA-molecule according to claim 16, **characterized in that** it is operatively linked to a transcription signal.

18. DNA-molecule according to claim 17, **characterized in that** the transcription signal is a promotor of a bacteriophage e.g. a T7 or SP6-promotor.

19. Cell containing a virus according to any one of the claims 1 to 13, a nucleocapsid according to claim 14, a genome according to claim 15 or a DNA-molecule according to any one of the claims 16 to 18.

20. Cell according to claim 19, **characterized in that** it is a vector-amplificating-cell.

21. Cell according to claim 19, **characterized in that** it is a virus-producing-cell.

22. Cell according to claim 21, **characterized in that** it furthermore contains a DNA-molecule encoding for a heterologous DNA-dependant RNA-polymerase, which provokes the transcription of the DNA-molecule encoding for the recombinant negative-strand RNA-virus.

23. Cell according to claim 19, **characterized in that** it is a virus-amplificating-cell.

24. Cell according to any one of the claims 19 to 23, **characterized in that** it furthermore contains DNA-molecules encoding for the viral P-protein.

25. Method for the production of a negative-strand RNA-virus according to any one of the claims 1 to 14, comprising the steps of:
(a) providing a cell which is transfected with a DNA-molecule encoding for the genome of a negative-strand RNA-virus, containing a mutation in the P-gene which leads to the loss of the viral genome-replicability without the loss of secondary transcription-capability, and, if necessary, at least one sequence encoding for a heterologous gene product, and
(b) cultivating the cell under conditions that a transcription of the DNA according to (a) takes place and the recombinant negative-strand RNA-virus is produced.

26. Method according to claim 25, furthermore comprising the extraction of the nucleocapsid or of the viral genome from the negative-strand RNA-virus.

27. Method for the amplification of a negative-strand RNA-virus according to any one of the claims 1 to 13, comprising the steps of:
(a) providing a cell which is infected with a negative-strand RNA-virus, containing a mutation in the P-gene which leads to the loss of the viral genome replicability without the loss of secondary transcription-capability, and, if necessary, at least one sequence encoding for a heterologous gene product, and
(b) cultivating the cell under conditions that an amplification of the virus takes place.

28. Pharmaceutical composition, **characterized in that** it contains a recombinant negative-strand RNA-virus according to any one of the claims 1 to 13, a nucleocapsid according to claim 14 or a viral genome according to claim 15 as an active agent, as well as, if necessary, conventional pharmaceutical carriers and/or adjuvants.

29. Pharmaceutical composition according to claim 28 for the use as a vaccine.

30. Pharmaceutical composition according to claim 29 as a mono or polyvalent vaccine.

31. Pharmaceutical composition according to claim 29 or 30 as a vaccine against viral infections, for example as a vaccine against infections with pathogenic negative-strand RNA-viruses.

32. Pharmaceutical composition according to claim 28 for the use in anti-tumor-therapy.

33. Pharmaceutical composition according to any one of the claims 28 to 32 for the use in risk-patients.

34. Pharmaceutical composition according to any one of the claims 28 to 33 comprising the nucleocapsid in the native viral envelope.

35. Use of a cell which constitutively or inducibly expresses stably the protein P of a negative-strand RNA-virus for the production or amplification of recombinant negative-strand RNA-viruses according to any one of the claims 1 to 13, of nucleocapsides according to claim 14 or of viral genomes according to claim 15.

36. Use according to claim 35, **characterized in that** the cell is a mammalian cell.

37. Use according to claim 35 or 36, **characterized in that** the cell is selected from the cell H29 (DSM ACC2702) or a cell derived therefrom.

## Revendications

1. Virus à ARN à brin négatif recombinant contenant un génome viral comportant une mutation dans le gène P qui entraîne la perte de la capacité de réplication sans perte de la capacité de transcription secondaire.

2. Virus selon la revendication 1,
**caractérisé en ce**
**qu'**il s'agit d'un paramyxovirus.

3. Virus selon la revendication 1 ou 2,
**caractérisé en ce**
**qu'**il s'agit d'un virus Sendaï.

4. Virus selon la revendication 1,
**caractérisé en ce**
**que** la mutation concerne la séquence partielle N-terminale de la protéine codant pour le gène P.

5. Virus selon la revendication 4,
**caractérisé en ce**
**que** la mutation comprend : une délétion
(a) des acides aminés 2 à 77 de la protéine codant pour le gène P ou
(b) d'une séquence partielle suffisante pour la perte de la capacité de réplication de (a).

6. Virus selon l'une quelconque des revendications 1 à 5,
**caractérisé en ce**
**que** le génome viral contient au moins une séquence codant pour un produit de gène hétérologue.

7. Virus selon la revendication 6,
**caractérisé en ce**
**que** le produit de gène hétérologue est une protéine, un ribozyme, une molécule anti-sens ou une molécule d'ARNsi.

8. Virus selon la revendication 6 ou 7,
**caractérisé en ce**
**que** le produit de gène hétérologue est une protéine reporter, un antigène ou une protéine thérapeutique.

9. Virus selon l'une quelconque des revendications 6 à 8,
**caractérisé en ce**
**que** le produit de gène est un antigène d'un agent pathogène hétérologue choisi parmi les virus, les bactéries et les protozoaires.

10. Virus selon l'une quelconque des revendications 6 à 9,
**caractérisé en ce**
**que** le produit de gène hétérologue est un antigène viral.

11. Virus selon la revendication 10,
**caractérisé en ce**
**que** le génome viral code pour plusieurs antigènes hétérologues des mêmes virus ou de virus différents.

12. Virus selon l'une quelconque des revendications 6 à 11,
**caractérisé en ce**
**que** la séquence codant pour au moins un produit de gène hétérologue est insérée dans le génome viral et/ou est substituée par une séquence codant pour un produit de gène homologue.

13. Virus selon l'une quelconque des revendications 1 à 12,
**caractérisé en ce**
**que** le virus présente par rapport au type sauvage, une capacité de transcription réduite au plus d'un facteur 20.

14. Nucléocapside d'un virus à ARN à brin négatif selon l'une quelconque des revendications 1 à 13.

15. Génome d'un virus à ARN à brin négatif selon l'une quelconque des revendications 1 à 13.

16. Molécule d'ADN qui code pour le génome et/ou l'anti-génome d'un virus à ARN à brin négatif recombinant selon l'une quelconque des revendications 1 à 13.

17. Molécule d'ADN selon la revendication 16,
**caractérisée en ce**
**qu'**elle se trouve en liaison fonctionnelle avec un signal de transcription.

18. Molécule d'ADN selon la revendication 17,
**caractérisée en ce**
**que** le signal de transcription est un promoteur de bactériophages, par exemple, un promoteur T7 ou SP6.

19. Cellule qui contient un virus selon l'une quelconque des revendications 1 à 13, un nucléocapside selon la revendication 14, un génome selon la revendication 15 ou une molécule d'ADN selon l'une quelconque des revendications 16 à 18.

20. Cellule selon la revendication 19,
**caractérisée en ce**
**qu'**elle est une cellule de multiplication de vecteur.

21. Cellule selon la revendication 19,
**caractérisée en ce**
**qu'**il s'agit d'une cellule de production de virus.

22. Cellule selon la revendication 21,
**caractérisée en ce**
**qu'**elle contient en outre une molécule d'ADN codant pour une ARN polymérase dépendante de l'ADN hétérologue qui entraîne la transcription de la molécule d'ADN codant pour le virus à ARN à brin négatif recombinant.

23. Cellule selon la revendication 19,
**caractérisée en ce**
**qu'**il s'agit d'une cellule de multiplication de virus.

24. Cellule selon l'une quelconque des revendications 19 à 23,
**caractérisée en ce**
**qu'**elle contient en outre une molécule d'ADN codant pour la protéine P virale.

25. Procédé de production d'un virus à ARN à brin négatif selon l'une quelconque des revendications 1 à 14, comprenant les étapes consistant à :
(a) se munir d'une cellule qui est transfectée avec une molécule d'ADN qui code pour le génome d'un virus à ARN à brin négatif, contenant une mutation dans le gène P qui entraîne la perte de la capacité de réplication du génome viral sans perte de la capacité de transcription secondaire et éventuellement au moins une séquence codant pour un produit de gène hétérologue, et
(b) cultiver la cellule dans des conditions telles qu'une transcription de l'ADN s'effectue selon le point (a) et le virus à ARN à brin négatif recombinant étant formé.

26. Procédé selon la revendication 25,
comprenant en outre la récupération du nucléocapside ou du génome viral à partir du virus à ARN à brin négatif.

27. Procédé de multiplication d'un virus à ARN à brin négatif selon l'une quelconque des revendications 1 à 13, comprenant les étapes consistant à :
(a) se munir d'une cellule qui est infectée par un virus à ARN à brin négatif, contenant une mutation dans le gène P qui entraîne la perte de la capacité de réplication du génome viral sans perte de la capacité de transcription secondaire et éventuellement au moins une séquence codant pour un produit de gène hétérologue, et
(b) cultiver la cellule dans des conditions telles qu'une multiplication du virus s'effectue.

28. Composition pharmaceutique,
**caractérisée en ce**
**qu'**elle contient un virus à ARN à brin négatif recombinant selon l'une quelconque des revendications 1 à 13, un nucléocapside selon la revendication 14 ou un génome viral selon la revendication 15 comme principe actif et éventuellement, des véhicules et/ou adjuvants classiques pharmaceutiquement acceptables.

29. Composition pharmaceutique selon la revendication 28 à utiliser comme vaccin.

30. Composition pharmaceutique selon la revendication 29 en tant que vaccin mono- ou polyvalent.

31. Composition pharmaceutique selon la revendication 29 ou 30, comme vaccin contre les infections virales, par exemple comme vaccin contre les infections par des virus à ARN à brin négatif pathogènes.

32. Composition pharmaceutique selon la revendication 28, à utiliser en traitement anti-tumoral.

33. Composition pharmaceutique selon l'une quelconque des revendications 28 à 32 à utiliser chez des patients à risque.

34. Composition pharmaceutique selon l'une quelconque des revendications 28 à 33, comprenant le nucléocapside dans l'enveloppe virale native.

35. Utilisation d'une cellule qui exprime de façon constitutive ou inductible stable, la protéine P d'un virus à ARN à brin négatif, pour la production ou la multiplication de virus à ARN à brin négatif recombinants selon l'une quelconque des revendications 1 à 13, de nucléocapsides selon la revendication 14 ou de génomes viraux selon la revendication 15.

36. Utilisation selon la revendication 35,
**caractérisée en ce**
**que** la cellule est une cellule de mammifère.

37. Utilisation selon la revendication 35 ou 36,
**caractérisée en ce**
**que** la cellule est choisie parmi la cellule H29 (DSM ACC2702) ou une cellule dérivée de celle-ci.
